# EUROPEAN PATENT SPECIFICATION

(11) **EP 4 140 440 B1**
(45) Date of publication and mention of the grant of the patent: **11.12.2024**
(21) Application number: 22795488.0
(22) Date of filing: 30.03.2022
(51) Int. Cl.: A61C 8/00, A61C 13/34

(54) **MODEL FOR DENTAL TREATMENT PLAN PRE-PROCEDURE VERIFICATION, DENTAL TREATMENT PLAN PRE-PROCEDURE VERIFICATION INSTRUMENT, MANUFACTURING METHOD FOR MODEL FOR DENTAL TREATMENT PLAN PRE-PROCEDURE VERIFICATION, DENTAL TREATMENT PLAN PRE-PROCEDURE VERIFICATION SYSTEM, AND DENTAL TREATMENT PLAN PRE-PROCEDURE VERIFICATION PROGRAM**
MODELL FÜR ZAHNVORBEHANDLUNGSPLANÜBERPRÜFUNG, ZAHNVORBEHANDLUNGSPLANÜBERPRÜFUNGSINSTRUMENT, HERSTELLUNGSVERFAHREN FÜR EIN MODELL FÜR DIE ZAHNVORBEHANDLUNGSPLANÜBERPRÜFUNG, ZAHNVORBEHANDLUNGSPLANÜBERPRÜFUNGSSYSTEM UND ZAHNVORBEHANDLUNGSPLANÜBERPRÜFUNGSPROGRAMM
MODÈLE POUR PRÉ-VÉRIFICATION DE PROCÉDURE DE PLAN DE TRAITEMENT DENTAIRE, INSTRUMENT DE PRÉ-VÉRIFICATION DE PROCÉDURE DE PLAN DE TRAITEMENT DENTAIRE, PROCÉDÉ DE FABRICATION DE MODÈLE POUR PRÉ-VÉRIFICATION DE PROCÉDURE DE PLAN DE TRAITEMENT DENTAIRE, SYSTÈME DE PRÉ-VÉRIFICATION DE PROCÉDURE DE PLAN DE TRAITEMENT DENTAIRE, ET PROGRAMME DE PRÉ-VÉRIFICATION DE PROCÉDURE DE PLAN DE TRAITEMENT DENTAIRE

(30) Priority: 30.04.2021 JP 2021077789
(43) Date of publication of application: 01.03.2023
(73) Proprietor: Lookin Co., Ltd., Tokyo 105-0003 (JP)
(72) Inventor: YOSHIHASHI, Noriaki, Tokyo 105-0003 (JP)
(74) Representative: AWA Sweden AB
(86) International application number: PCT/JP2022/015838
(87) International publication number: WO 2022/230572

(56) References cited:
- WO-A1-2019/243233
- JP-A- 2018 122 113
- KR-A- 20200 084 982
- KR-B1- 101 333 886
- US-A- 5 556 278
- US-A1- 2012 046 914
- US-A1- 2020 315 751

## Description

### FIELD

The present invention relates to a model for pre-procedure verification of a dental treatment plan, a pre-procedure verification instrument of a dental treatment plan, a method of manufacturing a model for pre-procedure verification of a dental treatment plan, a pre-procedure verification system of a dental treatment plan, and a pre-procedure verification program of a dental treatment plan.

### BACKGROUND

In an implant treatment for example, a dentist who is an operator, usually obtains a three-dimensional image of the upper jaw and/or the lower jaw, including a treatment position of the patient, using dental CT scanning. Based on the three-dimensional image, the dentist grasps, prior to the procedures, the position of the maxillary sinus and the positions of the posterior superior alveolar artery and the greater palatine artery of the upper jaw of the patient, or the positions of the inferior alveolar artery and the inferior alveolar nerve of the lower jaw of the patient, as well as the treatment position.

If the treatment objective is the upper jaw in an implant treatment for example, the dentist performs procedures in such a manner that the distal end of a hole-forming instrument such as a drill does not reach predetermined not-to-be-reached (not-to-be-contacted) targets, such as the maxillary sinus mucosa, the posterior superior alveolar artery, and the greater palatine artery of the patient. If the treatment objective is the lower jaw, the dentist performs procedures in such a manner that the distal end of the hole-forming instrument such as a drill does not reach predetermined not-to-be-reached targets, such as the inferior alveolar artery and the inferior alveolar nerve of the patient. US 2020/315751 A1 relates to a 3D printed bone supported sinus guide for edentulous maxillary arch.

### CITATION LIST

### PATENT LITERATURE

Patent Literature 1: Jpn. Pat. Appln. PCT Application No. 2017-508595
Patent Literature 2: U.S. Patent Application Publication No. 2012/0046914
Relevant prior art is exemplified by US 2020/315751 A1, US 5 556 278 A, KR 101 333 886 B1.

### SUMMARY

### TECHNICAL PROBLEM

The risk of implant treatment lies in, for example, that positions of not-to-be-reached targets such as arteries and nerves cannot be visually recognized during procedures.

The present invention aims at providing a model for pre-procedure verification of a dental treatment plan, a pre-procedure verification instrument of the dental treatment plan, a method of manufacturing a model for the pre-procedure verification of the dental treatment plan, a pre-procedure verification system of the dental treatment plan, and a pre-procedure verification program of the dental treatment plan which allow a dentist who is an operator, to perform, when performing, for example, a treatment of embedding an implant body, a treatment of embedding an embedded body such as an autologous tooth, etc., advance verification of a positional relationship between a position of a distal end of a procedural instrument during use of the procedural instrument and positions of not-to-be-reached targets of a patient such as positions of the maxillary sinus mucosa, the posterior superior alveolar artery, and the greater palatine artery of the upper jaw or positions of the inferior alveolar artery and the inferior alveolar nerve of the lower jaw, prior to a dental treatment using the procedural instrument such as an actual hole-forming instrument, after creating the treatment plan.

### SOLUTION TO PROBLEM

According to one aspect of the present invention, there is provided a model for pre-procedure verification of a dental treatment plan. The model includes a model main body, an opening defining portion, and a target model portion. The model main body is formed based on a jaw of a patient in a size and a shape identical to at least a part of a treatment objective site of the jaw of the patient and configured to model the at least a part of the treatment objective site. The opening defining portion is provided in the model main body and is configured to define an opening corresponding to a recessed opening for embedding an embedded body in the treatment objective site. The target model portion is configured to model a target adjacent to or embedded in an alveolar bone of the treatment objective site. The target model portion allows a distal end of a procedural instrument used for a treatment to be out of contact with the target model portion under a condition that the procedural instrument passes through the opening defining portion in a positional relationship of the target model portion identical to a positional relationship of the target relative to the treatment objective site, or allows the distal end of the procedural instrument to be in contact with the target model portion under a condition that the procedural instrument passes through the opening defining portion in a positional relationship of the target model portion closer than the positional relationship of the target relative to the treatment objective site. A space is formed between the opening defining portion and the target model portion to allow visual recognition of a range present between the opening defining portion and the target model portion. The range including a reachable range of the distal end of the procedural instrument from the opening defining portion toward the target model portion and a reachable range of the distal end of the procedural instrument from the opening defining portion in a teeth alignment direction and a direction intersecting the teeth alignment direction.

### BRIEF DESCRIPTION OF THE DRAWINGS

FIG. 1 is a block diagram of a dental treatment plan advance verification system according to a first embodiment and a second embodiment.
FIG. 2 is a schematic diagram showing an example of an implant body.
FIG. 3 is a schematic diagram showing an example of a hole-forming instrument.
FIG. 4A is a schematic diagram showing a model for advance verification of a dental treatment plan according to the first embodiment.
FIG. 4B is a schematic diagram showing a model as viewed from a direction different from that of FIG. 4A.
FIG. 4C is a schematic diagram showing a model for advance verification of a dental treatment plan according to a modification of the first embodiment.
FIG. 5 is a schematic diagram showing a surgical guide according to the first embodiment.
FIG. 6 is a flowchart showing a treatment plan of an implant treatment.
FIG. 7 is a flowchart showing the treatment plan of the implant treatment subsequent to FIG. 6.
FIG. 8 is a diagram showing a state in which a third three-dimensional image on which positions of not-to-be-reached targets are marked is superimposed on a first three-dimensional image showing the inferior alveolar bone.
FIG. 9 is a diagram showing a state in which a fourth three-dimensional image of an implant body is further superimposed on FIG. 8.
FIG. 10 is a diagram showing a state in which data of a second three-dimensional image of teeth and gums and a fifth three-dimensional image of the hole-forming instrument are superimposed on FIG. 9.
FIG. 11 is a diagram showing a sixth three-dimensional image of a surgical guide.
FIG. 12 is a schematic diagram showing the gums, the teeth, the inferior alveolar bone, the implant body, and the not-to-be-reached targets, with the surgical guide attached to the lower jaw.
FIG. 13 is a schematic diagram showing the gums, the teeth, the inferior alveolar bone, the hole-forming instrument, and the not-to-be-reached targets, with the surgical guide attached to the lower jaw.
FIG. 14 is a diagram showing a state in which the sixth three-dimensional image of the surgical guide is further superimposed on FIG. 9.
FIG. 15 is a diagram showing a state in which the first three-dimensional image and the second three-dimensional image are removed from FIG. 14.
FIG. 16 is a diagram showing a surface image of parts of the second three-dimensional image, the third three-dimensional image, the fourth three-dimensional image, and the fifth three-dimensional image.
FIG. 17 is a diagram showing a surface image obtained by superimposing the surface image shown in FIG. 16 and then subtracting the fourth three-dimensional image and the fifth three-dimensional image therefrom.
FIG. 18A is a schematic diagram showing a state in which the surgical guide is combined with the model and then the hole-forming instrument is inserted into the guide opening of the surgical guide.
FIG. 18B is a schematic diagram showing a state in which the hole-forming instrument is inserted into the opening portion of the model.
FIG. 19 is a schematic diagram showing a state in which the second three-dimensional image and the third three-dimensional image are superimposed on the first three-dimensional image of the lower jaw, according to a second modification of the first embodiment.
FIG. 20 is a schematic diagram showing the gum, the teeth, the inferior alveolar bone, a hole-forming instrument including a handle, and not-to-be-reached targets, with a surgical guide attached to the lower jaw, according to a first modification.
FIG. 21 is a diagram showing a state in which a third three-dimensional image on which positions of not-to-be-reached targets are marked, a fourth three-dimensional image of an autologous tooth, and a fifth three-dimensional image of a hole-forming instrument are superimposed on a first three-dimensional image showing the inferior alveolar bone, according to a second modification.
FIG. 22 is a diagram showing a state in which a fourth three-dimensional image of an implant body and a fifth three-dimensional image of a hole-forming instrument are superimposed on a first three-dimensional image showing the superior alveolar bone, the maxillary sinus, the posterior superior alveolar artery, and the greater palatine artery of the upper jaw, according to a second embodiment.
FIG. 23 is a diagram showing a state in which first to fourth three-dimensional images of the upper jaw are superimposed.
FIG. 24 is a diagram showing a state in which sixth three-dimensional image data is superimposed on the diagram shown in FIG. 23, and the first three-dimensional image is removed therefrom.
FIG. 25 is a diagram showing surface image data of parts of the first three-dimensional image, the second three-dimensional image, the third three-dimensional image, the fourth three-dimensional image, and the fifth three-dimensional image.
FIG. 26 is a diagram showing a surface image obtained by superimposing the surface image data shown in FIG. 25 and then subtracting the fourth three-dimensional image and the fifth three-dimensional image therefrom.
FIG. 27 is a diagram of the upper jaw shown in FIG. 26 as viewed from a different direction.
FIG. 28 is a schematic diagram showing a positional relationship between the teeth and the maxillary sinus during procedures of embedding an implant body in the upper jaw.
FIG. 29 is a schematic diagram showing a positional relationship between the teeth and the maxillary sinus during procedures of embedding an implant body in the upper jaw, subsequent to FIG. 28.
FIG. 30 is a diagram showing a state in which the maxillary sinus floor mucosa of the maxillary sinus is superimposed on the second three-dimensional image in FIG. 23.

FIG. 31 is a schematic diagram showing a state of a series of processes leading to a three-dimensional image of a model of the upper jaw, according to a modification of a second embodiment.

### DETAILED DESCRIPTION

A pre-procedure verification system (model production system for verifying a dental treatment plan prior to procedures after creation of the dental treatment plan) 10 of a dental treatment plan according to an embodiment forms part of a series of operations from, for example, acquisition of various types of data of a patient and creation of a treatment plan of an implant treatment to performance of procedures on the patient. The system 10 is used in performing a series of operations from acquisition of patient data of a patient, creation of a dental treatment plan for the patient, to performance of pre-procedure verification of the dental treatment plan using an output of the model (model for pre-procedure verification of the dental treatment plan) 100 of the actual patient. The acquisition of patient data of a patient and the output of the model 100 may be performed by a system different from the system 10. The model 100 according to the present embodiment is a real-size model.

### (First Embodiment)

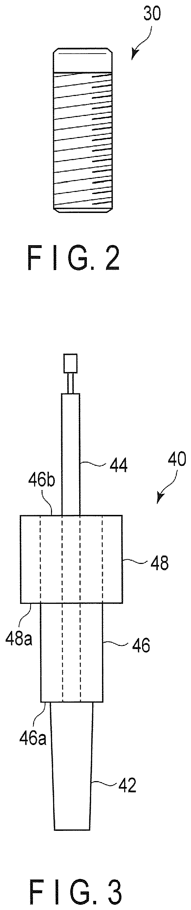

In the first embodiment, a case will be described where a system 10 is used in an example of performing an implant treatment in which an implant body 30 (see FIG. 2), selected from a multitude, is embedded as an embedded body in the lower jaw of the patient.

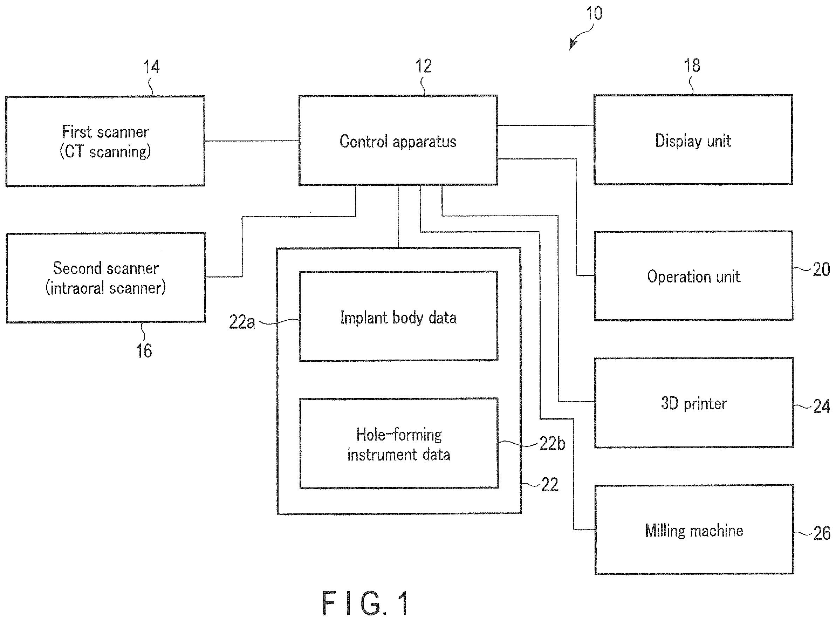

As shown in FIG. 1, a pre-procedure verification system (hereinafter simply referred to as a "system") 10 of a dental treatment plan includes a control apparatus 12, a first scanner 14, a second scanner 16, a display 18, an operation portion (instruction input portion) 20, a storage apparatus 22, a 3D printer 24, and a milling machine 26.

The control apparatus 12 controls the first scanner 14, the second scanner 16, the display 18, the operation portion 20, the storage apparatus 22, the 3D printer 24, and the milling machine 26. Examples of the control apparatus 12 that may be used include a computer. The control apparatus 12 includes, for example, a processor such as a CPU and an MPU as well as a RAM, a ROM, and an I/O interface. The control apparatus 12 causes one or more processors such as CPUs in a memory such as a ROM to develop a control program stored in a memory such as a ROM into a RAM, and execute suitable processing on the display 18, the operation portion 20, the storage apparatus 22, the first scanner 14, the second scanner 16, the 3D printer 24, and the milling machine 26. Alternatively, the control apparatus 12 causes one or more processors such as CPUs to read a program via a network, and execute suitable processing on the display 18, the operation portion 20, the storage apparatus 22, the first scanner 14, the second scanner 16, the 3D printer 24, and the milling machine 26. The control apparatus 12 causes the processor to read and execute programs stored in a memory in advance to control each component, thereby realizing the function of performing processing such as image processing by means of software.

The first scanner 14 is based on, for example, dental CT scanning. The first scanner 14 acquires a three-dimensional image (e.g., DICOM data) of, for example, the teeth, the bone, and the inside of the bone of the lower jaw of the patient, and outputs it to the control apparatus 12. The control apparatus 12 causes the storage apparatus 22 to store the three-dimensional image of the teeth, the bone, and the inside of the bone of the lower jaw of the patient. The second scanner 16 is, for example, an intraoral scanner. The second scanner 16 acquires, for example, a three-dimensional image (e.g., STL data) of the surface of the teeth and the gums of the lower jaw of the patient, and outputs it to the control apparatus 12. The control apparatus 12 causes the storage apparatus 22 to store the three-dimensional image of the surface of the teeth and the gums of the lower jaw of the patient.

The display 18 is, for example, a variety of displays such as a liquid crystal display and an organic electroluminescence (EL) display. The display 18 displays a patient-related image acquired by the first scanner 14 and the second scanner 16 to be output to the control apparatus 12 and displays a variety of information. The control apparatus 12 may read a three-dimensional image of the patient stored in the storage apparatus 22 and cause the display 18 to display the three-dimensional image.

The operation portion 20 inputs an instruction to the control apparatus 12. The operation portion 20 includes, for example, a device such as a keyboard and a mouse.

The storage apparatus 22 stores various types of data on a patient (e.g., a three-dimensional image (e.g., DICOM data) of the teeth, the bone, and the inside of the bone of the lower jaw, and a three-dimensional image (e.g., STL data) of the surface of the teeth and the gums of the lower jaw of the patient. The storage apparatus 22 stores, for example, a variety of three-dimensional images (implant body data) 22a of an implant body 30 shown in FIG. 2, and a variety of three-dimensional images (hole-forming instrument set data) 22b of a hole-forming instrument 40 such as a drill shown in FIG. 3.

If the control apparatus 12 reads, via a network, a variety of three-dimensional images 22a of the implant body 30 and a variety of three-dimensional images 22b of the hole-forming instrument 40, the storing of the variety of three-dimensional images 22a of the implant body 30 and the variety of three-dimensional images 22b of the hole-forming instrument 40 in the storage apparatus 22 may be eliminated. That is, the control apparatus 12 may use, for example, a database (the implant body data 22a and the hole-forming instrument set data 22b) on a server instead of the storage apparatus 22. The control apparatus 12 is configured to read a variety of data from the server.

As shown in FIG. 3, the actual hole-forming instrument 40 includes, for example, a body 42 that bores a hole, a shank 44, a sleeve 46, and a stopper 48. The body 42 and the shank 44 are made integral by, for example, integral molding. The shank 44 is fixed to an unillustrated handpiece. This allows the body 42 and the shank 44 to be rotated around a predetermined rotation axis. The sleeve 46 covers the outer side of the body 42, and fits into or engages with a guide opening (through opening) 210 of an adjunctive instrument 200 such as a surgical guide, to be described later. An end surface 48a on the side of the body 42 of the stopper 48 abuts a defining surface 220 which defines the guide opening 210 of the adjunctive instrument 200, with a distance to the handpiece being defined, for example.

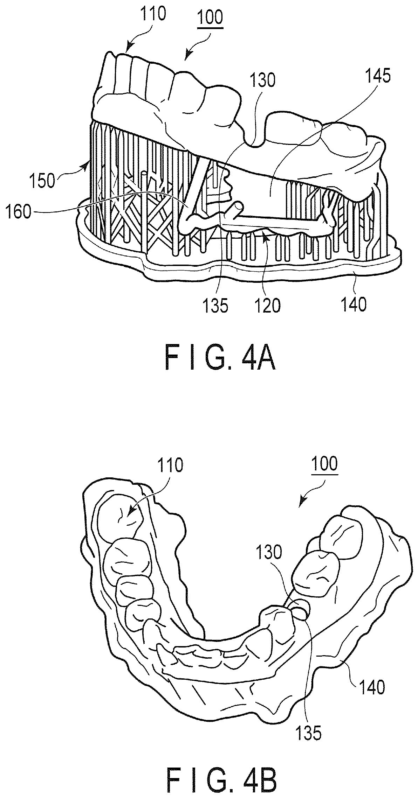

The 3D printer 24 constructs a model 100 for advance verification of a dental treatment shown in FIGS. 4A and 4B, based on a three-dimensional image of the lower jaw of the patient and a three-dimensional image created by a dental treatment planner such as a dentist. It is preferable, for example, that the 3D printer 24 be owned by a dentist himself/herself who operates the 3D printer 24; however, a professional, etc. may receive data for the 3D printer from a dentist, etc. and output a construction.

It is preferable that the 3D printer 24 according to the present embodiment be formed to automatically construct, at the time of output, a support material (a base portion (foundation) 140 and one or more pillars 150 to be described later), together with a model main body 110 in which an opening portion (opening defining portion) 130 is provided, and a target model portion 120. A dentist, etc. may suitably place a support material in a fifth three-dimensional image 55, to be described later, prior to the output by the 3D printer 24.

The model 100 includes a model main body 110, a target model portion 120, and an opening portion (opening defining portion) 130. The model main body 110 is constructed to have the same size and shape as those of the treatment objective site and its periphery of the actual lower jaw of the patient. The model main body 110 need not cover the entire lower jaw, and should only cover the treatment objective site and its periphery of the lower jaw. It is preferable that the model main body 110 be formed to cover the same range as the region in which an adjunctive instrument (surgical guide) 200, to be described later, is fixed to the treatment objective site and its periphery of the actual lower jaw of the patient, or is formed as a greater surface model. It is preferable that much of the site corresponding to the inferior alveolar bone of the patient not be present in the model 100. The model main body 110 and the target model portion 120 are formed to have the same positional relationship as the positional relationship between the treatment objective site and a target (a not-to-be-contacted target or a to-be-contacted target) of the actual lower jaw of the patient. The same positional relationship means that the site relating to procedures of the treatment objective site is formed to have the same size and shape as the treatment objective site and its periphery of the actual lower jaw of the patient. The opening portion 130 is defined by a variety of parameters of the implant body 30 and the hole-forming instrument 40, which will be described later. The opening portion 130 is formed in accordance with settings of parameters relating to the shape (e.g., the length, the outer diameter, etc.), the angle, and the position of the implant body 30 or the hole-forming instrument 40 to be embedded in the opening portion 130 of the treatment objective site.

The model main body 110 has a defining surface 135 that forms an edge of the opening portion 130. The defining surface 135 is a surface of the gums or the inferior alveolar bone. If an end surface 46a of the sleeve 46 of the hole-forming instrument 40 abuts the defining surface 135, the body 42 of the hole-forming instrument 40 is restricted from going toward the back side of the gums and the inferior alveolar bone from that position. Thus, a distal end reaching position of the hole-forming instrument 40 is defined by the defining surface 135 of the model main body 110.

It is preferable that the target model portion 120 be supported on the model main body 110 with a main pillar 160. A space (space defining portion) 145 is formed between the opening portion 130 and the target model portion 120 to allow the dentist, etc. to visually recognize, from outside the model 100, a reachable range of the distal end of the hole-forming instrument 40 from the opening portion 130 toward the target model portion 120, and the range within which the distal end of the hole-forming instrument 40 may reach from the opening portion 130 in a teeth alignment direction and a direction intersecting the teeth alignment direction. That is, the space 145 is formed between the model main body 110 and the target model portion 120 to allow a dentist, etc. to confirm whether a distal end of the body 42 of the hole-forming instrument 40 is, for example, in contact with or out of contact with the target model portion 120. In the present embodiment, the main pillar 160 and the target model portion 120 are formed as an approximately L-shaped member. The main pillar 160 is formed as a part of a frame that defines the space 145, together with the model main body 110, the target model portion 120, etc.

The model 100 further includes a base portion (foundation) 140 provided on a side opposite to the model main body 110 to support the target model portion 120, and one or more pillars (supports) 150 connecting the model main body 110 and the base portion 140.

It is preferable that a position corresponding to the cheek side and/or the lip side of the alveolar bone between the model main body 110 and the target model portion 120 be accessible to the target model portion 120 from the side of the position corresponding to the cheek side and/or the lip side of the alveolar bone as a wall-less window portion.

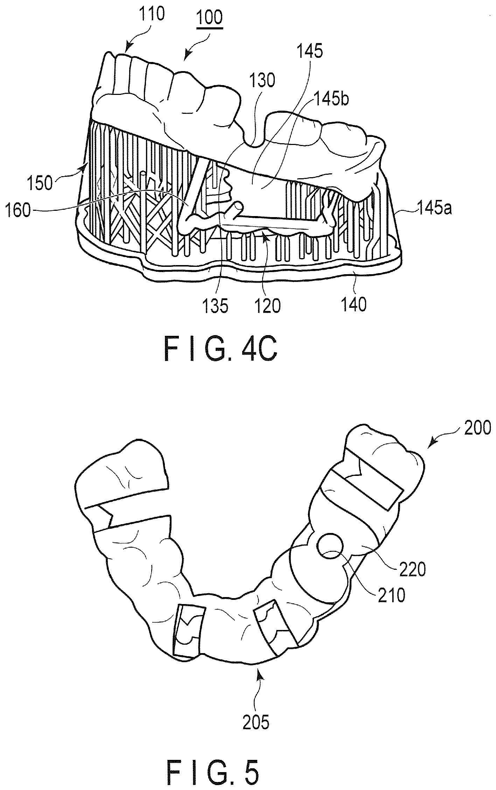

The milling machine 26 carves the adjunctive instrument (surgical guide) 200 shown in FIG. 5 based on the three-dimensional image of the lower jaw of the patient and the three-dimensional image created by the dentist. It is preferable that the milling machine 26 be owned by a dentist himself/herself who operates the milling machine 26; however, a professional, etc. may receive data for the milling machine 26 from a dentist, etc. and output the adjunctive instrument 200.

For actual use during a dental treatment, a medically approved resin material having a durability that withstands the dental treatment is used as the adjunctive instrument 200. The adjunctive instrument 200 used during a dental treatment may be produced by the 3D printer 24. In this case, the adjunctive instrument 200 is formed of a medically approved material. It is preferable that the adjunctive instrument 200, which is used for the dentist's confirmation together with the model 100, not be used during a dental treatment, and is formed of, for example, the same material as that of the model 100. The model 100, which is not used for an actual treatment, may be formed of a suitable material with a suitable precision, as long as the relationship between the defining surface 135 and the target model portion 120 is maintained.

The adjunctive instrument 200 is formed to conform to the surface shape of a three-dimensional image of the surface of the teeth and the gums and/or a CT image of a treatment portion of the patient. The adjunctive instrument 200 is used by being fixedly screwed to, and then fitted into, the teeth in the vicinity of the gums of the treatment portion, the gums, or the jawbone of the patient. The adjunctive instrument 200 is fixed to the lower jaw so as not to wobble in the lower jaw. The adjunctive instrument 200 is used to form a recessed opening set in the gums and the alveolar bone, and to precisely form a recessed opening for receiving the set implant body 30 in the body 42 of the hole-forming instrument 40.

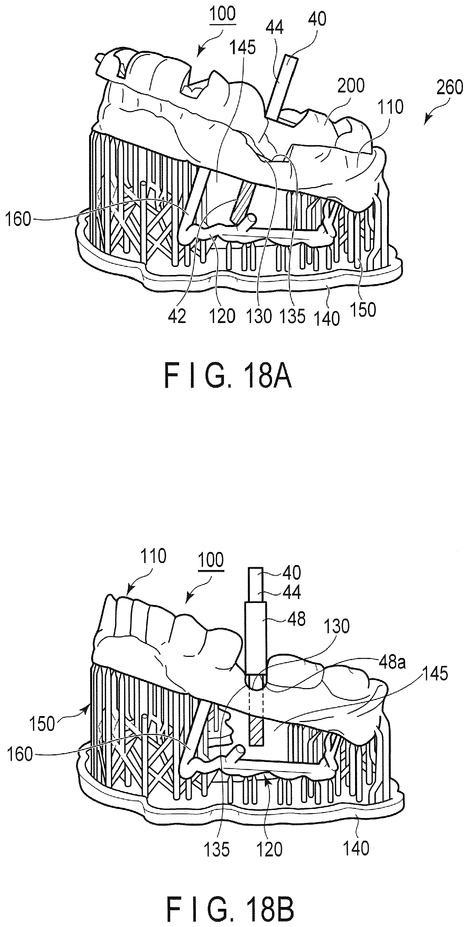

The adjunctive instrument 200 is used together with the model 100 for pre-procedure verification for a dental treatment plan, as well as during a dental treatment. As shown in FIG. 18A, a combination of the model 100 for pre-procedure verification of a dental treatment plan and the adjunctive instrument 200 is referred to as a pre-procedure verification instrument 260 of a dental treatment plan.

The adjunctive instrument 200 shown in FIG. 5 includes a main body 205 and a guide opening 210.

Since the adjunctive instrument 200 is used by being fixedly screwed to, and then fitted into the teeth in the vicinity of the gums of the treatment portion, the gum, or the jawbone of the patient, as described above, the main body 205 is used as a position defining portion that defines a referential position relative to the teeth, the gums, and the jawbone. An example is shown in which the main body 205 is fixed along one or more teeth in the oral cavity region of the patient; however, there may be completely no teeth on the lower jaw side of the patient. The main body 205 may be configured, for example, to be connected to a smaller part of the oral cavity of the patient, such as to only one or two teeth, only a bone, or a given combination thereof.

With the main body 205 appropriately attached to the teeth and the gums of the lower jaw of the patient, the guide opening 210 defines the direction, the shape (the length and the outer diameter), the angle, the position, etc. of a recessed opening formed by the body 42 of the hole-forming instrument 40. The adjunctive instrument 200 includes a defining surface 220 that forms an edge of the guide opening 210. The defining surface 220 is a surface on a side opposite to a surface that faces the gums or the inferior alveolar bone. If an end surface 48a of the stopper 48 abuts the defining surface 220, the body 42 of the hole-forming instrument 40 is restricted from going toward the back side of the gums and the inferior alveolar bone from that position. Thus, a distal end reaching position of the hole-forming instrument 40 is defined by the defining surface 220 of the adjunctive instrument 200.

It is to be noted that not only the hole-forming instrument 40 but also other procedural instruments such as an electrosurgical knife for hemostasis, for example, may be inserted into the guide opening 210 of the adjunctive instrument 200. At this time, the defining surface 220 restricts the direction, the angle, the position, etc. of insertion of the electrosurgical knife. Thus, the defining surface 220 may be used not only as a restricting surface of the hole-forming instrument 40, but also as a restricting surface of other procedural instruments. Accordingly, the adjunctive instrument 200 is used as a position defining body of the procedural instrument. The guide opening 210 is usually formed as a circular opening.

It is to be noted that, with the system 10 according to the present embodiment, a dentist is capable of creating the adjunctive instrument 200 as a three-dimensional image (data representing a three-dimensional shape), and constructing an actual object that fits the shape and size of the lower jaw of the patient, using the 3D printer 24 or the milling machine 26.

In the storage apparatus 22 according to the present embodiment, an image display program, an image processing program, an output program, etc. are stored. The image display program, the image processing program, the output program, etc. are executed by the control apparatus 12.

The image display program allows data acquired by the first scanner 14 and the second scanner 16 to be displayed on the display 18. The image processing program superimposes an image (image data) displayed by the image display program and a variety of data stored in the storage apparatus 22 based on an identical coordinate axis. The image display program allows the data obtained by the superimposition based on the identical coordinate axis using the image processing program to be displayed on the display 18. The image display program allows a three-dimensional image created based on the dentist's intention to be displayed on the display 18. The output program outputs surface data (which refers to data (three-dimensional image data) representing a three-dimensional shape, and will be hereinafter referred to as "surface data") of three-dimensional images and objects (e.g., the model 100 and the adjunctive instrument 200) created based on the dentist's intention using the image processing program. The output program is configured to output surface data that allows the 3D printer 24 or the milling machine 26 to construct an actual object.

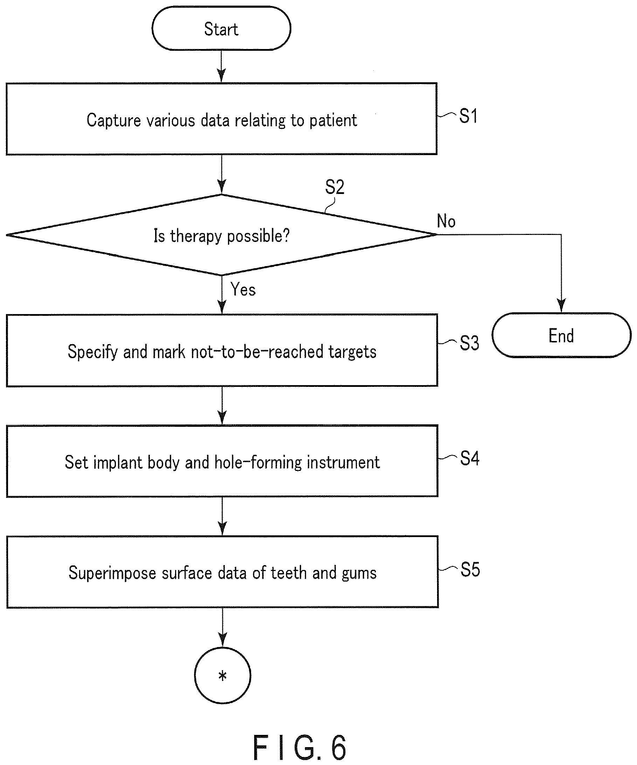

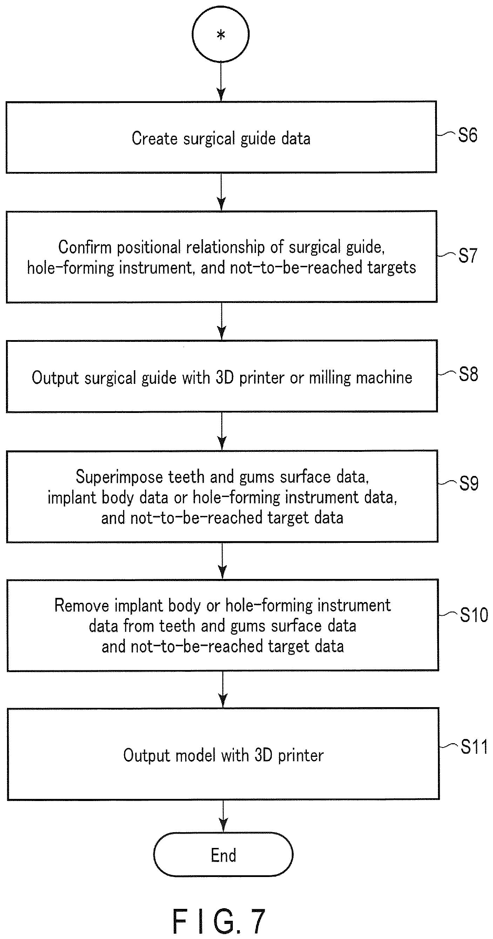

The dentist inputs a variety of instructions to the control apparatus 12 using the operation portion 20, and creates a treatment plan of an implant treatment in accordance with, for example, the flows shown in FIGS. 6 and 7.

The control apparatus (computer) 12 acquires a three-dimensional image (e.g., DICOM data) of the teeth, the bone, and the inside of the bone of the lower jaw of the patient using, for example, the first scanner 14, which is based on dental CT scanning or the like, and causes the storage apparatus 22 to store the acquired three-dimensional image. This is referred to as a first three-dimensional image (three-dimensional image data) 51. Also, the control apparatus (computer) 12 acquires a three-dimensional image (e.g., STL data) of the surface of the teeth and the gums using the second scanner 16, which is, for example, an intraoral scanner, and causes the storage apparatus 22 to store the acquired three-dimensional image. This is referred to as a second three-dimensional image (three-dimensional image data) 52, namely, first surface data. The second three-dimensional image 52 (first surface data) includes a treatment objective site and its peripheral site. The control apparatus (computer) 12 imports the first three-dimensional image 51 and the second three-dimensional image 52 in a suitable piece of software (an application) based on the dentist's instruction (step S1). In the software, data of both the first three-dimensional image 51 and the second three-dimensional image 52 can be read, even if the first three-dimensional image 51 and the second three-dimensional image 52 are in different data formats.

It is to be noted that the "dentist's instruction" in the present embodiment includes various examples such as simply clicking a computer mouse.

The dentist confirms, for example, the first three-dimensional image 51 on a display screen of the display 18, and comprehensively judges whether or not it is possible to perform an implant treatment on a treatment site of a patient based on various other conditions (step S2). Hereinafter, an operation in the case where the dentist has judged that an implant treatment is possible (step S2-Yes) will be described. It is to be noted that, if the dentist has judged that an implant treatment is impossible (step S2-No), an operation of creating a treatment plan ends.

It is to be noted that, if the second three-dimensional image 52 is not used for judging whether or not an implant treatment is possible, acquisition of a three-dimensional image of the surface of the teeth and the gums using the second scanner 16 may be performed after step S2.

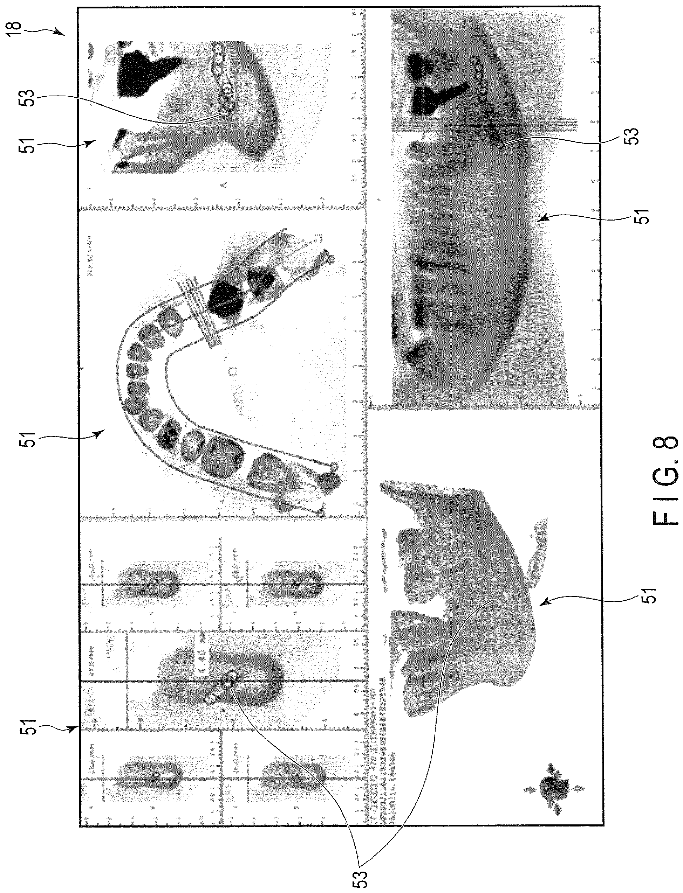

As shown in FIG. 8, based on the dentist's instruction, the control apparatus 12 specifies, on a software-based screen of the display 18, the positions of the inferior alveolar artery and the inferior alveolar nerve in the first three-dimensional image 51 indicating the bone (inferior alveolar bone) of the lower jaw. The positions of the inferior alveolar artery and the inferior alveolar nerve are, for example, not-to-be-reached (not-to-be-contacted) targets that must not be reached by a distal end of the body 42 of the hole-forming instrument 40 during formation of a recessed opening 70 into which an implant body 30 is to be embedded during an implant treatment. Based on the dentist's instruction, the control apparatus 12 marks, on the screen of the display 18, the positions of the not-to-be-reached targets (step S3; first process). At this time, based on the dentist's instruction, the control apparatus 12 marks, on the screen of the display 18, characteristic points of the not-to-be-reached targets in such a manner that the not-to-be-reached targets are three-dimensionally formed. Based on the dentist's instruction, the control apparatus 12 creates a three-dimensional image 120a of the not-to-be-reached targets as a third three-dimensional image (fourth surface data) 53. The third three-dimensional image 53 includes a three-dimensional image 160a of the main pillar 160. That is, the third three-dimensional image 53 includes a three-dimensional image 120a of a not-to-be-reached target and a three-dimensional image 160a of the main pillar 160. Thus, specifying a target includes specifying a three-dimensional image 160a of the main pillar 160 joined to the second three-dimensional image 52 (image based on the first surface data). The three-dimensional image 160a of the main pillar 160 of the model 100 is formed by making a mark at a position that does not become an obstacle in the case where the dentist brings the distal end of the hole-forming instrument 40 close to the target model portion 120 through the opening portion 130.

The three-dimensional image 160a of the main pillar 160 may be configured in such a manner that, after the three-dimensional image 100a of the model 100 is created, a three-dimensional image 160a of the main pillar 160 for the joining may be set to maintain the positional relationship between the three-dimensional image 110a of the model main body 110 and the three-dimensional image 120a of the target model portion 120.

It is to be noted that, if, for example, the three-dimensional image 160a of the main pillar 160 is automatically created by, for example, the function of the 3D printer 24, the specification of the three-dimensional image 160a may be eliminated.

Based on the dentist's instruction, the control apparatus 12 may specify, in the first three-dimensional image 51, the position of a lingual-side surface (wall) of the inferior alveolar bone close to the not-to-be-reached targets such as the inferior alveolar artery and the inferior alveolar nerve. Based on the dentist's instruction, the control apparatus 12 may mark, on the screen of the display 18, the position of the lingual-side surface as a not-to-be-reached target of the third three-dimensional image 53.

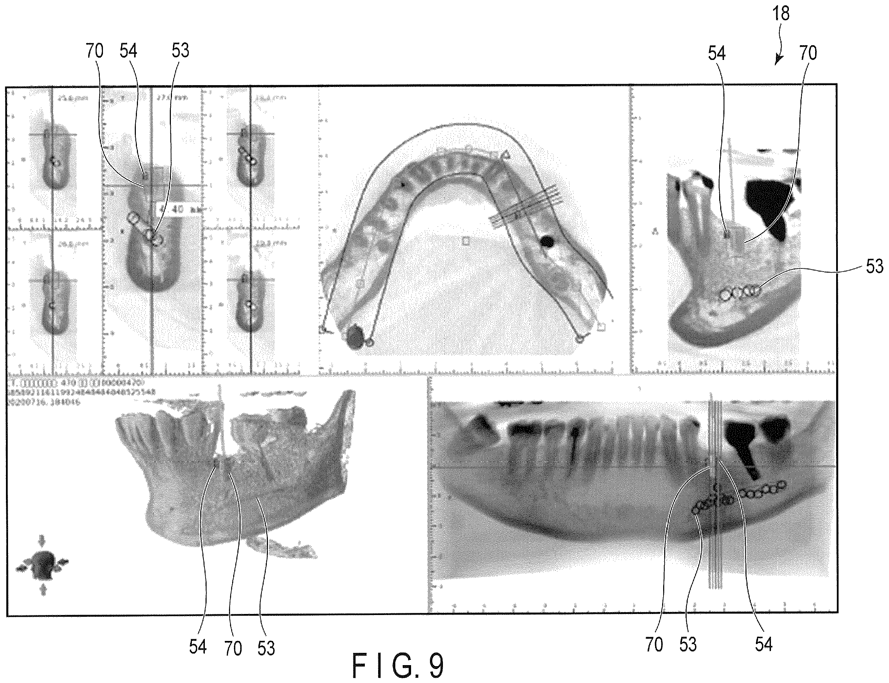

Based on the dentist's instruction, the control apparatus 12 sets or installs, on the screen of the display 18, the shape (e.g., the length and the outer diameter), the angle, the position, etc. of an implant body that models the implant body 30 at the treatment position, as shown in FIG. 9 (step S4). The term "installation" refers to importing three-dimensional data acquired by, for example, a dentist using a 3D scanner into the system 10. The term "angle of the implant body" refers to, for example, the installation orientation relative to the treatment position of the patient.

It is to be noted that, for the implant body, three-dimensional image data corresponding one-to-one to the implant body and containing the same shape and the same dimensional information as that of the actual implant body 30 that has been actually medically approved is used. At this time, the dentist takes into consideration the relationship with the shapes of an abutment and an upper structure (crown part) to be attached to the implant body 30. The dentist selects, for example, a single implant body that matches the settings from among a plurality of three-dimensional images 22a of implant bodies stored in the storage apparatus 22. A fourth three-dimensional image (second surface data) 54 of the implant body selected by the dentist is provided by, for example, the manufacturer of the implant body 30. The dentist may create the fourth three-dimensional image 54 of the implant body by himself/herself if such an image is not provided by the manufacturer of the implant body 30. The control apparatus 12 causes the storage apparatus 22 to store the fourth three-dimensional image 54 of the implant body created by the dentist.

The dentist can select the implant body 30 by himself/herself, and set the angle, the position, etc. of the implant body 30 relative to the bone of the lower jaw to be optimum according to the patient. At this time, it is easy for the dentist to select the implant body again and test fitness of implant bodies with different lengths and outer diameters on the treatment objective site of the patient.

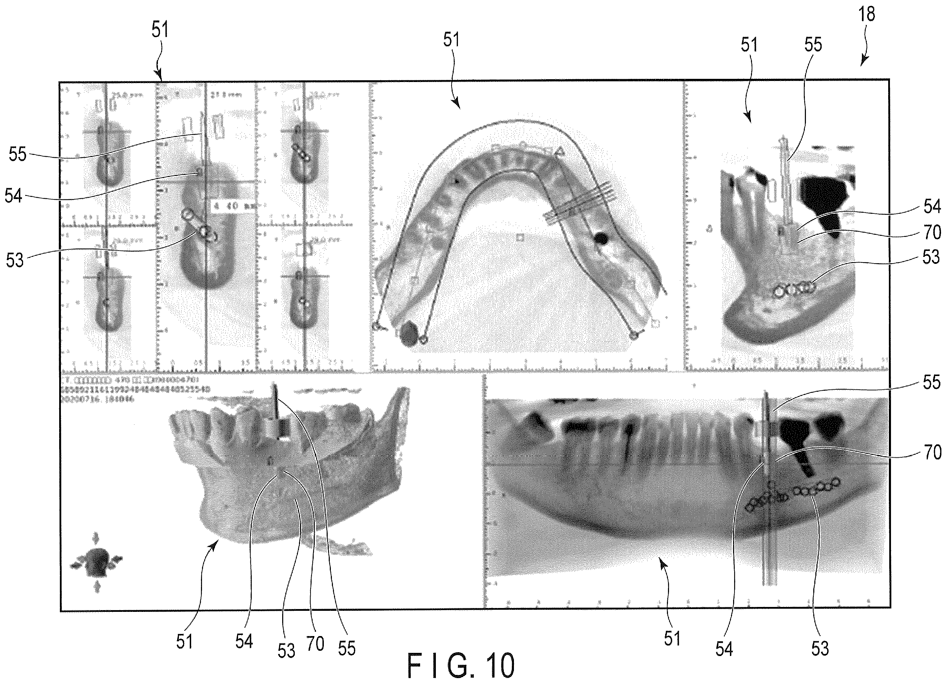

Based on the dentist's instruction, the control apparatus 12 selects, from among a plurality of drill sets of one or more manufacturers used for patients' treatment, an appropriate hole-forming instrument according to the depth, the diameter, the angle, and the position of the recessed opening 70 for embedding the selected implant body, as shown in FIG. 10 (second processing). It is to be noted that, for the hole-forming instrument, a three-dimensional image corresponding one-to-one to the hole-forming instrument and containing information on the same shape and the same dimensions as those of the hole-forming instrument 40 that has been actually medically approved is used. The dentist may usually select a hole-forming instrument 40 recommended by the manufacturer of the selected implant body 30; however, a hole-forming instrument 40 of a manufacturer other than the manufacturer of the selected implant body 30 may be used. Based on the dentist's instruction, the control apparatus 12 selects a single hole-forming instrument 40 that matches the settings from a plurality of three-dimensional images 22b of the hole-forming instrument that models the hole-forming instrument 40 stored in the storage apparatus 22. A fifth three-dimensional image (third surface data) 55 of the hole-forming instrument 40 selected by the control apparatus 12 is provided by, for example, the manufacturer of the hole-forming instrument 40. If the fifth three-dimensional image 55 is not provided by the manufacturer of the hole-forming instrument 40, the dentist may create the fifth three-dimensional image 55 by himself/herself. The control apparatus 12 causes the storage apparatus 22 to store the fifth three-dimensional image 55 created by the dentist.

It is to be noted that, in general, the diameter of the opening portion 130 formed by the actual hole-forming instrument 40 is slightly smaller than the outer diameter of the actual implant body 30. Such a relationship remains the same on the screen of the display 18. A difference in outer diameter between the hole-forming instrument and the implant body can be suitably set as a parameter input by the dentist to the operation portion 20.

Based on the dentist's instruction, the control apparatus 12 aligns, on the software-based screen of the display 18, coordinate axes of the first to fifth three-dimensional images 51-55, and superimposes the second three-dimensional image (e.g., STL data) 52 on the first three-dimensional image (e.g., DICOM data) 51, the third three-dimensional image 53, the fourth three-dimensional image 54, and/or the fifth three-dimensional image 55, in a predetermined coordinate system in which the coordinate axes are aligned (step S5). At this time, the dentist finds, for example, points of identity in size and shape, such as the alignment of teeth, and performs matching between the first three-dimensional image 51 and the second three-dimensional image 52. Such matching may be performed automatically by the control apparatus 12 using software. The first three-dimensional image 51 includes a third three-dimensional image 53, a fourth three-dimensional image 54, and/or a fifth three-dimensional image 55. Thus, the control apparatus 12 may clearly show the positional relationship between the surface of the teeth and the gums of the lower jaw (the second three-dimensional image 52) and the arteries and the nerves of the inside of the alveolar bone (the third three-dimensional image 53) on the display 18, as shown in FIG. 10, using software. Thus, the control apparatus 12 may clearly show the positional relationship among the surface of the teeth and the gums of the lower jaw, the alveolar bone, the not-to-be-reached targets, the implant body, and the hole-forming instrument to the dentist using software.

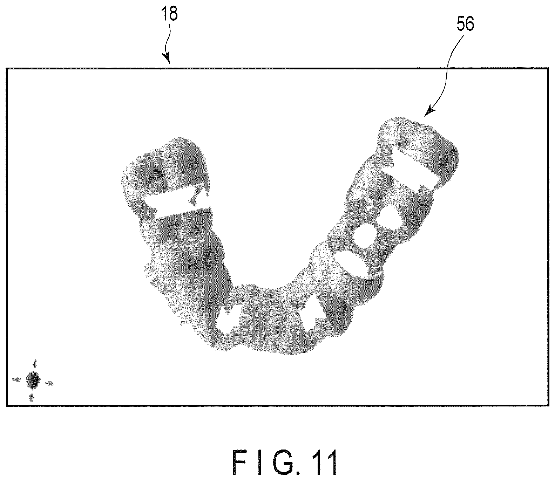

Based on the dentist's instruction, the control apparatus 12 creates, on the software-based screen of the display 18, a three-dimensional image of the adjunctive instrument (surgical template) in accordance with the shape of the lower jaw of the patient, as shown in FIG. 11 (step S6). That is, the control apparatus 12 creates image data of the adjunctive instrument as a sixth three-dimensional image 56.

The adjunctive instrument 200 shown in FIG. 5 guides the body 42 of the hole-forming instrument 40 along the treatment portion, while covering a portion of the surface of the gum. In determining a treatment plan based on the dentist's instruction, the control apparatus 12 outputs a three-dimensional image (e.g., STL data) for the 3D printer 24 or the milling machine 26 of the adjunctive instrument 200, and causes the 3D printer 24 or the milling machine 26 to construct the adjunctive instrument 200. The adjunctive instrument 200 can be fit into or engages with the model 100 (see FIGS. 4A and 4B) created by the 3D printer 24. It is to be noted that a resin material used for the adjunctive instrument 200 differs according to whether or not the adjunctive instrument 200 is actually used during a treatment. If a medically approved resin material is used as the adjunctive instrument 200, the adjunctive instrument 200 can be used as it is. If a medically non-approved resin material is used as the adjunctive instrument 200, the adjunctive instrument 200 cannot used for an actual treatment as it is. In this case, the adjunctive instrument 200 fit into the model 100 may be used for pre-procedure verification of a created treatment plan to confirm, for example, that the distal end of the hole-forming instrument 40 is placed at a predetermined position, and that the target model portion 120 is not contacted.

In actual procedures, the dentist cannot confirm how many millimeters have been dug from the surface of the gums (not the alveolar bone) during formation of a recessed opening in the treatment objective site of the patient. However, the dentist can confirm in advance how many millimeters have been dug from the surface of the alveolar bone, and a distance from the surface of the alveolar bone to the artery, using tools to be actually used.

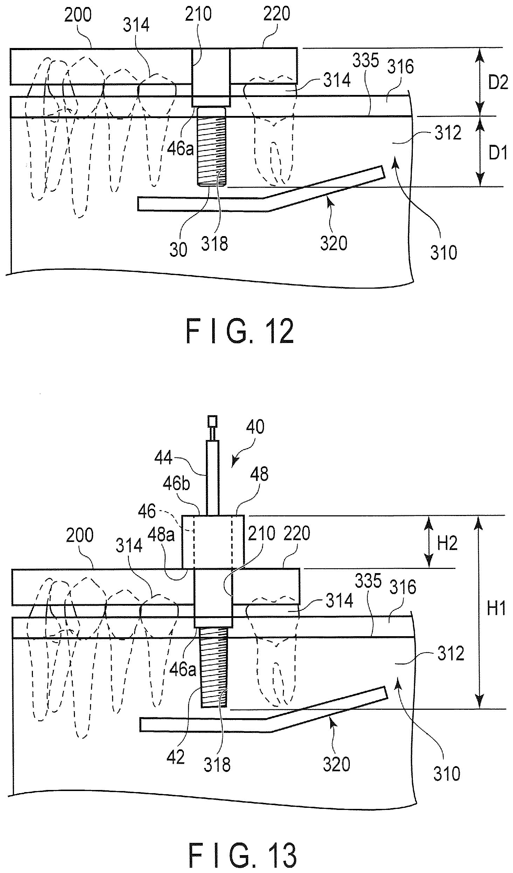

FIGS. 12 and 13 show a schematic diagram of the lower jaw 310, including the alveolar bone 312, the teeth 314, the gums 316, the recessed opening 318, and the not-to-be-reached targets 320 including the inferior alveolar artery and the inferior alveolar nerve.

As shown in FIG. 12, in an actual implant treatment, the dentist forms the recessed opening 318 in the inferior alveolar bone 312 and the gums 316 using, for example, the adjunctive instrument 200 and the hole-forming instrument 40. The recessed opening 318 has a depth equal to the sum of a distance D1 from a top of the inferior alveolar bone 312 to a bottom of the recessed opening 318 and a distance (offset value) D2 from a top (defining surface) 335 of the inferior alveolar bone 312 to a defining surface 220 of the adjunctive instrument 200. That is, in the case of using the adjunctive instrument 200, the depth of the recessed opening 318 is offset from the top of the inferior alveolar bone 312 to the defining surface 220 of the adjunctive instrument 200.

In this case, as shown in FIGS. 12 and 13, the dentist selects the hole-forming instrument 40 and the implant body 30 in such a manner that (length H1 of drill body 42 below upper end 46b of drill sleeve 46)-(height H2 of stopper 48)=(length D1 of implant body 30 used during surgery)+(offset value D2 during use of adjunctive instrument 200) is satisfied. The dentist forms the recessed opening 318 in such a manner that a bottom of the recessed opening 318 or a bottom of the implant body 30 and the not-to-be-reached targets 320 are separated from each other, and embeds the implant body 30 into the recessed opening 318. That is, the dentist suitably inputs the above-described parameters H1, H2, D1, and D2 through the use of the operation portion 20, using the shape (e.g., outer diameter), the position, and the angle of the implant body 30 or the inner diameter, the position, and the angle of the recessed opening 70 as parameters, and creates an optimum treatment plan while confirming the state relative to the treatment objective site of the patient. The setting or installation of the parameters H1 and H2 includes selection of an optimum hole-forming instrument 40 by the dentist.

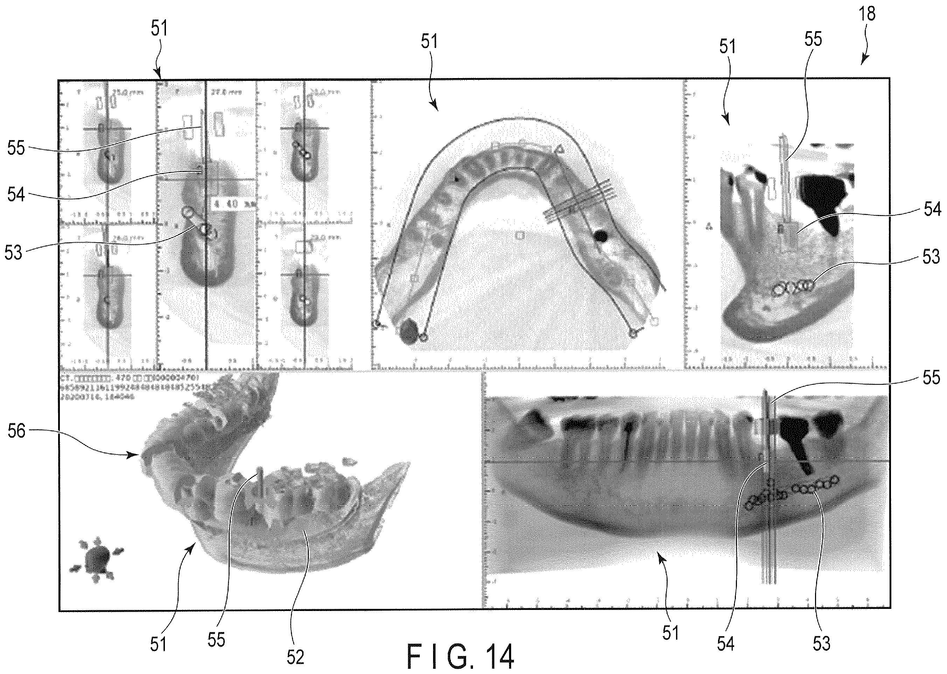

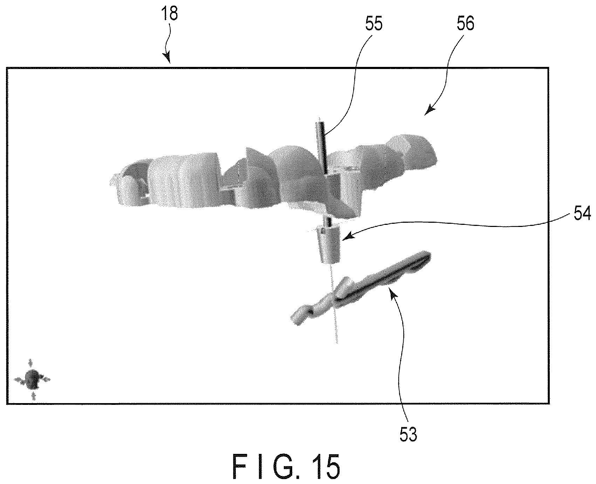

Based on the dentist's instruction, the control apparatus 12 superimposes, on a predetermined coordinate system, the first three-dimensional image 51 of the bone of the lower jaw, the second three-dimensional image 52 of the surface of the teeth and the gum, the third three-dimensional image 53 of the not-to-be-reached targets, the fourth three-dimensional image 54 of the implant body, and/or the fifth three-dimensional image 55 of the hole-forming instrument, and the sixth three-dimensional image 56 of the adjunctive instrument, as shown in FIG. 14 (step S7, fifth process). The dentist confirms, on the display 18, a state of placement of, for example, the sixth three-dimensional image 56, the fourth three-dimensional image 54 or the fifth three-dimensional image 55, and the third three-dimensional image 53, as shown in FIG. 15. That is, the control apparatus 12 clearly shows, on the software-based screen of the display 18, a positional relationship between the adjunctive instrument, the implant body, the hole-forming instrument for forming an opening for embedding an implant body, and the not-to-be-reached targets to the dentist.

It is to be noted that the control apparatus 12 performs the processing from step S3 to step S7 using the image processing program, and causes the display 18 to perform display using the image display program.

The control apparatus 12 constructs the adjunctive instrument 200 by means of the 3D printer 24 or the milling machine 26 (step S8).

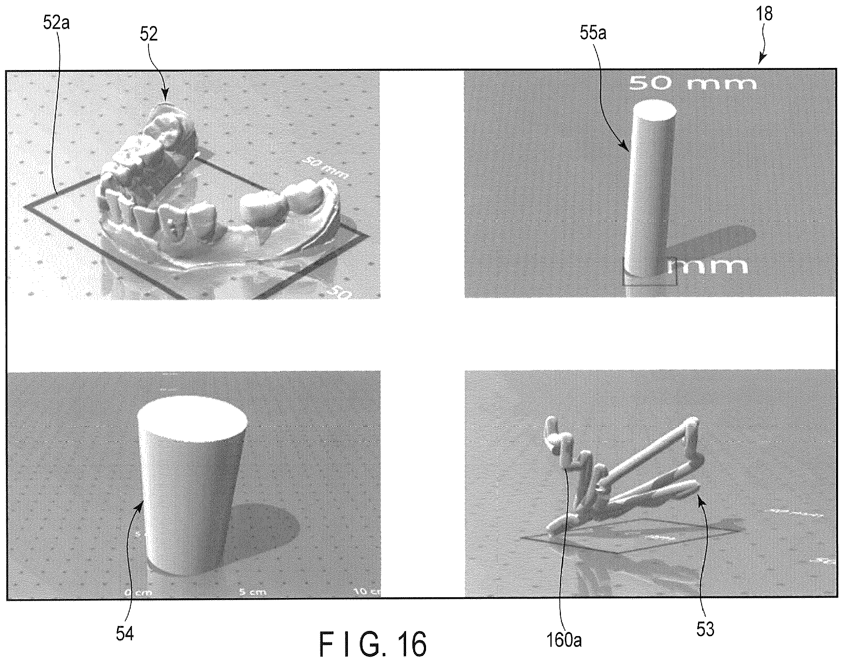

The dentist confirms whether or not there is any problem that should be fixed in the treatment plan on the software-based screen of the display 18 of the control apparatus 12. If there is any problem, the problem is fixed. If there is no problem, based on the dentist's instruction, the control apparatus 12 superimposes, on a predetermined coordinate system, the second three-dimensional image 52, the third three-dimensional image 53, the fourth three-dimensional image 54, and the fifth three-dimensional image 55 (step S9), as shown in FIG. 16. In the second three-dimensional image 52, by deleting an image portion of a site opposite to the defining surface 135 or the teeth of the surface of the gums, bordered by a suitable plane 52a, for example, the lingual-side and cheek-side walls are removed, with the gums surface including the defining surface 135 of the teeth-side gums and the teeth maintained. It is to be noted that, depending on the second three-dimensional image 52 that can be obtained by the second scanner 16, partial deletion of the image bordered by the plane 52a can be eliminated.

At this time, based on the dentist's instruction, the control apparatus 12 superimposes, on a predetermined coordinate system on the screen of the display 18, the second three-dimensional image 52, the third three-dimensional image 53, the fourth three-dimensional image 54, and the fifth three-dimensional image 55 by means of software that creates a treatment plan. Alternatively, if the three-dimensional images are of compatible data types, the control apparatus 12 may, based on the dentist's instruction, import the three-dimensional images into, for example, 3D CAD software different from the software that creates the treatment plan, and superimpose the three-dimensional images. At this time, the control apparatus 12 imports only an image (sleeve-related fifth three-dimensional image) 55a of a portion that does not include a body and a shank assumed to be used for procedures, for example, a sleeve (guide tube), of the fifth three-dimensional image 55 of the hole-forming instrument.

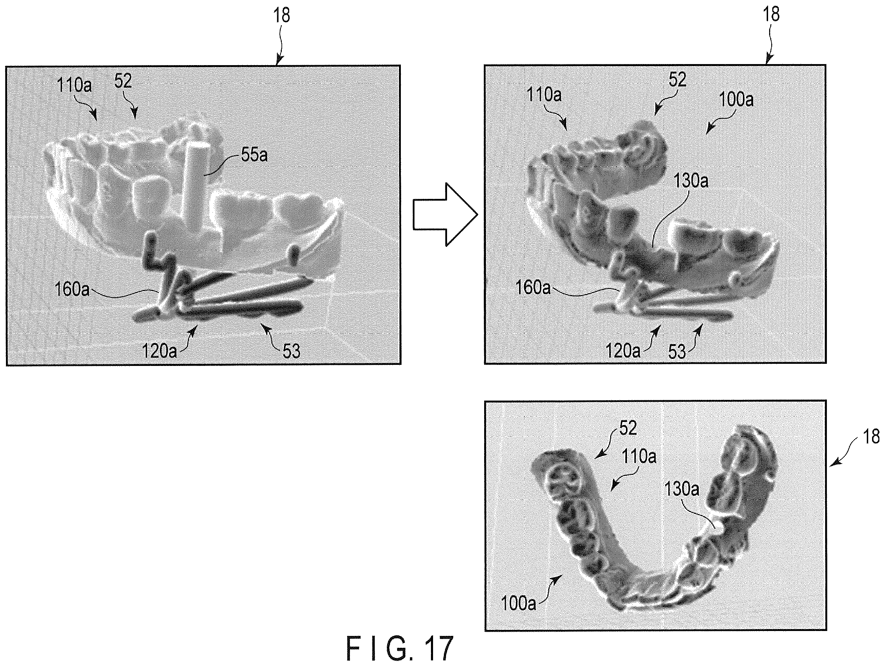

Based on the dentist's instruction, the control apparatus 12 removes, on the software, the fourth three-dimensional image 54 relating to the implant body and the fifth three-dimensional image 55a relating to the sleeve (step S10, third process), as shown in FIG. 17. That is, the control apparatus 12 creates, on the software-based screen of the display 18, a three-dimensional image (fifth surface data) 100a of the model 100 including a three-dimensional image 110a of the model main body 110 including a through opening 130a that models a recessed opening for embedding a tooth or an implant body, and a three-dimensional image 120a of the target model portion 120 for the 3D printer 24.

It is preferable that a three-dimensional image corresponding to the support (the base portion 140 and the pillars (sub-pillars) 150) be automatically created using the function of the 3D printer 24.

The control apparatus 12 performs the processing from step S9 to step S10 using the image processing program, and causes the display 18 to perform display using the image display program.

The dentist confirms the three-dimensional image 100a of the model 100, which is data for the 3D printer 24. After that, the control apparatus 12 outputs the three-dimensional image (fifth surface data) 100a of the model 100 to, for example, the 3D printer 24. That is, the dentist constructs, under an operation input instruction to the operation portion 20, the model 100 (see FIGS. 4A and 4B) (step S11, fourth process) of the treatment site of the patient including a target model portion 120 that models the inferior alveolar artery and the gums including a through opening 130 that models a recessed opening 70 for embedding a tooth or the implant body 30 with the 3D printer 24 controlled by the control apparatus 12 (step S11, fourth process).

Here, the dentist temporarily ends the treatment plan creation process using the system 10.

In this manner, the operation portion 20 of the above-described system 10 gives, to the control apparatus 12, a processing instruction to specify a treatment objective site of the patient and not-to-be-reached targets in a three-dimensional stereo image of the patient, and a processing instruction to set an opening that models a recessed opening for embedding an implant body to be embedded in the treatment objective site by setting a variety of parameters of the implant body or installing data acquired by the dentist using a 3D scanner, etc. in a three-dimensional stereo image of the patient. Also, the operation portion 20 inputs, to the control apparatus 12, a processing instruction (coordinate conversion instruction) to superimpose first surface data (surface-image-related data), second surface data or third surface data, and fourth surface data on a predetermined coordinate system, and a processing instruction to create fifth surface data indicating a positional relationship between the fourth surface data and a treatment objective site including an opening by subtracting the second surface data and the third surface data from the first surface data.

That is, the operation portion 20 inputs, to the control apparatus 12, a processing instruction (coordinate conversion instruction) to superimpose the first surface data, at least one of the second surface data and the third surface data, and the fourth surface data on a predetermined coordinate system, and a processing instruction to create fifth surface data indicating a positional relationship between the fourth surface data and a treatment objective site including an opening by subtracting the second surface data and the third surface data from the first surface data, and the control apparatus 12 performs the instructed processing. It is to be noted that, if the second surface data is not used in the coordinate conversion instruction, the second surface data need not be subtracted during the creation of the fifth surface data. If third surface data is not used in the coordinate conversion instruction, the third surface data need not be subtracted during the creation of the fifth surface data.

The dentist fits, for example, an adjunctive instrument 200 suitable for the hole-forming instrument 40 and the shape of the gums of the treatment objective site of the patient into the model 100 constructed by the 3D printer 24, as shown in FIG. 18A. Furthermore, the dentist inserts the body 42 of the hole-forming instrument 40 into the guide opening 210 of the adjunctive instrument 200. At this time, the hole-forming instrument 40 uses the sleeve 46 and the stopper 48 in a manner similar to the actual procedures. The dentist visually confirms the positional relationship between the distal end of the body 42 of the hole-forming instrument 40 and the target model portion 120. Specifically, upon insertion of the hole-forming instrument 40, fit into the guide opening 210 of the adjunctive instrument 200, into the through opening 130 to bore a recessed opening for embedding the implant body 30 with the body 42 of the hole-forming instrument 40, the dentist confirms whether or not the body 42 of the hole-forming instrument 40 faces a desired direction, or the distal end of the body 42 maintains a state of being separated from the target model portion 120 of the arteries, the nerves, and the like.

It is to be noted that the dentist, etc. removes the foundation 140 and the pillars 150 from the model 100 as necessary, to confirm the state of visual recognition of the distal end of the body 42 of the hole-forming instrument 40.

The dentist confirms a range within which the distal end of the body 42 of the hole-forming instrument 40 can move. During the hole formation by the hole-forming instrument 40, the distal end of the body 42 of the hole-forming instrument 40 is moved so as not to break the lingual-side wall of the alveolar bone. Thus, the dentist confirms that the distal end of the body 42 of the hole-forming instrument 40 does not touch a membranous body 145b, which is the lingual-side wall of the model 100.

In an actual treatment, it is recommended to make the distal end of the body 42 of the hole-forming instrument 40 separate from the inferior alveolar nerve and the inferior alveolar artery by 3 mm or greater. Thus, a treatment plan may be created in such a manner that the target model portion 120 of each of the inferior alveolar nerve and the inferior alveolar artery is created so as to be greater than the actual one toward the defining surface 135 by, for example, 3 mm or greater, and that the distal end of the hole-forming instrument 40 abuts thereto. That is, by making a part of the target model portion 120 closer to the side of the defining surface 135 than the positions of the actual not-to-be-reached targets, the dentist can judge whether or not it is possible to use the hole-forming instrument 40 actually used for the patient, based on a positional relationship between the position at which the distal end of the body 42 of the hole-forming instrument 40 abuts the target model portion 120 and the end surface 48a on the side of the body 42 of the stopper 48 of the hole-forming instrument 40. That is, the dentist can also form, for example, the target model portion 120 as a to-be-reached (to-be-contacted) target, not as a not-to-be-reached (not-to-be-contacted) target.

In this manner, the dentist can perform advance verification of the procedure of creating a recessed opening in which the implant body 30 is to be embedded, using the hole-forming instrument 40 that is actually used, by constructing, using the system 10, a model 100 of the same size and shape as those of the actual patient, using the three-dimensional images 51 and 52 of the patient and the three-dimensional images 54 and 55 of the implant body and the hole-forming instrument. If there is no problem in the advance verification, the dentist performs procedures on the actual patient in accordance with the treatment plan. If a problem arises in the advance verification, the dentist fixes the treatment plan as necessary, re-creates the model 100 and the adjunctive instrument 200, and performs advance verification of the procedures. The dentist repeats the operation as necessary until there is no problem in advance verification.

It is to be noted that, in the case of constructing the model 100, the dentist may selectively use the three-dimensional images 54 and 55 of the implant body and the hole-forming instrument.

The position, size, angle, etc. of the through opening 130 of the model main body 110 are formed to have a final size for fitting the implant body 30. In actual procedures, the dentist digs the hole, which is originally small, to gradually increase the diameter and the depth of the hole. Thus, in formation of a recessed opening in actual procedures, the dentist advances the procedure by gradually changing the length and the diameter of the drill body 42 from smallest to largest. In the system 10, it is possible to describe what specific hole-forming instrument is used by the dentist to form a recessed opening as a treatment plan; however, in a three-dimensional image constructed by the 3D printer 24, a recessed opening of a final size that allows the implant body 30 to be embedded therein may be set.

By using a plurality of models 100 that fit the sizes and shapes of the respective drill bodies 42, the dentist can perform advance verification to advance procedures using the model 100 constructed by making the size of the through opening 130 conform to the drill diameter by gradually changing the drill length and diameter from smallest to largest. That is, by using the model 100, it is possible to perform advance verification of a variety of procedural instruments used in creation of the recessed opening, as well as the hole-forming instrument 40 that is finally used. Examples of the procedural instrument used during creation of the recessed opening include an injection instrument which injects a bone graft material, fibrin gel containing platelet, etc. separated from the withdrawn blood, or a mixture thereof into the treatment objective site.

At present, the dentist may perform actual procedures by calculating various parameters such as the length of the body 42, the height of the stopper 48, and offset values of the hole-forming instrument during the procedures in accordance with the implant body 30 to be used. If the dentist makes an error in any one of the parameters, an instrument different from the instrument that should be originally used might be used, possibly leading to a medical accident. By using the model 100 according to the present embodiment, the dentist himself/herself can perform advance verification as to the validity of the use of each procedural instrument in actual procedures until a final-size recessed opening is formed.

Also, the creator of the treatment plan in the system 10, such as a dentist, may make an error in various parameters such as the length of the body 42, the height of the stopper 48, and offset values of the hole-forming instrument 40 during creation of the treatment plan. There may be a case where the creator of the treatment plan misses an error in the parameters and ends the creation of the treatment plan. Even in such a case, by performing advance verification of a treatment plan using the model 100 and the actual hole-forming instrument 40, the dentist can notice setting faults in the parameters in the treatment plan. The dentist can discuss, using the model 100, how the parameters such as the length of the body 42, the height of the stopper 48, and offset values of the hole-forming instrument 40 to be used should be changed to perform the procedures successfully. In this case, a hole-forming instrument other than the hole-forming instrument 40 of the manufacturer set during the creation of the treatment plan can be suitably tried. Thus, by using the model 100, the dentist can appropriately select the hole-forming instrument 40 from among, for example, a plurality of instruments that the dentist owns.

Accordingly, advance verification of procedures by the dentist using the model 100, as described in the present embodiment, should be performed as an integral part of an implant treatment. Thus, through advance verification of procedures using the model 100, the dentist can appropriately change the hole-forming instrument 40 as necessary, and perform an optimum treatment. It is to be noted that the dentist may fix the treatment plan using the system 10, as a matter of course. Thus, through the use of the model 100 according to the present embodiment by the dentist, it is possible to greatly increase the safety of the implant treatment.

In an implant treatment, the dentist creates, for example, the adjunctive instrument 200, creates a recessed opening of a predetermined size at a desired position of the lower jaw of the patient, and embeds the implant body 30 into the recessed opening. Conventionally, due to the relationship between the hole-forming instrument 40 and the adjunctive instrument 200 for embedding the implant body 30, there has been no means for the dentist to visually and actually confirm, prior to procedures, whether or not a distal end of the body 42 of the hole-forming instrument 40 reaches the not-to-be-reached targets if an alternative hole-forming instrument of another manufacturer is selected based on the dentist's own idea. According to the present embodiment, the dentist can verify, prior to procedures, the relationship between a model main body 110 including the treatment objective site of the patient, the target model portion 120, the adjunctive instrument 200, and the hole-forming instrument 40 or the implant body 30, using the model 100 and the actual hole-forming instrument 40 or the implant body 30. That is, the dentist can perform advance verification of the created treatment plan prior to the actual procedures. This allows the dentist to perform actual procedures by performing advance verification of the safety of procedures with the hole-forming instrument 40. Since the dentist grasps in advance the distal end reaching position of the body 42 of the hole-forming instrument 40 relative to the not-to-be-reached targets, namely, a separation distance or an abutment state between the not-to-be-reached targets and the distal end of the body 42 of the hole-forming instrument 40 in the model 100, it is possible, in actual procedures, for the dentist to shorten the time required for the procedures. This allows the dentist to perform minimally invasive procedures on the patient by performing procedures in accordance with the treatment plan.

The dentist can perform a treatment plan creation process using the system 10 between a medical action of acquiring patient data using a first scanner (CT scanner) 14 and a second scanner (intraoral scanner) 16 and a medical action of actually performing procedures on the patient. In the present embodiment, an example has been described in which the dentist creates a treatment plan by himself/herself. The treatment plan creation process is an extremely important process leading to a medical action of performing procedures to embed the implant body 30 into the lower jaw, even though actual treatment and diagnosis of the patient is not performed. Thus, the process of creating, by the dentist, a treatment plan using the system 10 and performing advance verification of procedures using the model 100 created based on the treatment plan and the adjunctive instrument 200 is extremely effective for ensuring safety of the implant treatment. For that purpose, to ensure the safety of the treatment, it is extremely useful for the dentist himself/herself to use the system 10 configured to create an optimum treatment plan for each patient.

Since the creation of the treatment plan itself is not a direct medical action, as described above, there may be a case where a person who is unqualified for a dental treatment action such as a technician of the manufacturer or a dental mechanic creates a treatment plan. Even in such a case, the dentist can receive treatment plan creation data, and discuss a treatment plan and give a fixation instruction or perform fixation by himself/herself in the system 10. In either case, the dentist can perform advance verification of the safety of the treatment using the model 100, the hole-forming instrument 40, the implant body 30, and the adjunctive instrument 200 immediately before performing the actual procedures.

In the case of fixing the above-described treatment plan and re-creating the model 100, it is possible to reduce the time for correspondence with the professional such as the manufacturer by the dentist himself/herself performing a series of operations using the system 10. Thus, in the case where the dentist creates a treatment plan and outputs the model 100 with the 3D printer 24, it is possible to greatly reduce the time, for example, in units of weeks, compared to the case where the professional is used. Accordingly, the dentist can arrange a state in which procedures on the patient can be performed at an earlier stage.

In the case where the dentist uses the system 10, the dentist can take the initiative in laying an optimum treatment plan by trial and errors. Thus, even if the output of the adjunctive instrument 200 and the model 100 is left to the professional, the number of fixations of the adjunctive instrument 200 and the model 100 can be reduced. Accordingly, the dentist can arrange a state of performing procedures on the patient at an earlier stage.

It is to be noted that, conventionally, an adjunctive instrument (a surgical guide) has not necessarily been required. Thus, it is hard to say that, in an implant treatment as of now, adjunctive instruments are in widespread use. By the dentist himself/herself designing the adjunctive instrument 200 using the system 10 according to the present embodiment and outputting it using, for example, the 3D printer 24 or the milling machine 26 owned by the dentist himself/herself, it is possible to greatly reduce the expense for creation of the adjunctive instrument 200. Accordingly, by using the system 10 according to the present embodiment, it is possible to spread the use of adjunctive instruments such as a surgical guide across dentists during an implant treatment.

Accordingly, by using the system 10, it is possible to allow the dentist to take the initiative in creating a treatment plan as much as possible, thus reducing the cost for creating a treatment plan including the adjunctive instrument 200 and disseminating treatment with higher safety using the adjunctive instrument 200.

As described above, according to the present embodiment, it is possible to provide a pre-procedure verification system 10 of a dental treatment plan, a pre-procedure verification program of the dental treatment plan, a method of manufacturing a model 100 for pre-procedure verification of the dental treatment plan, and the model 100 for the pre-procedure verification of the dental treatment plan, which allow the dentist to perform, in performing a treatment of embedding an embedded body such as an implant body, advance verification of the positional relationship between the position of a distal end of a procedural instrument during use of the procedural instrument and target positions such as the positions of the inferior alveolar artery and the inferior alveolar nerve of the lower jaw of the patient, prior to a dental treatment using an actual procedural instrument such as a hole-forming instrument 40.

It is to be noted that, if the adjunctive instrument is not used, the hole-forming instrument 40 is inserted into the opening portion 130 of the model 100 constructed by the 3D printer 24, as shown in FIG. 18B. The model 100 is formed in such a manner that the end surface 48a on the side of the body 42 of the stopper 48 abuts the defining surface 135 of the model main body 110. The dentist confirms the range within which the distal end of the body 42 of the hole-forming instrument 40 can move, and visually confirms the positional relationship between the distal end of the body 42 of the hole-forming instrument 40 and the target model portion 120. Specifically, the dentist confirms, upon insertion of the body 42 of the hole-forming instrument 40 into the through opening 130 to bore a recessed opening for embedding the implant body 30, whether or not the body 42 of the hole-forming instrument 40 faces a desired direction, and whether or not the distal end of the body 42 maintains a state of separation from the target model portion 120 of the arteries, the nerves, etc.

In the model 100 shown in FIGS. 4A and 4B, there is no site corresponding to the inferior alveolar bone of the patient. There may be a site corresponding to the inferior alveolar bone as membranous bodies 145a and 145b that allows the dentist to visually recognize the target model portion 120, with the resin material of the model 100 created by the 3D printer 24 being, for example, transparent or semitransparent, as shown in FIG. 4C. The membranous body 145a models the cheek-side or lip-side wall of the alveolar bone. The membranous body 145a models the lingual-side wall of the alveolar bone. At this time, it is preferable that the distal end of the body 42 of the hole-forming instrument 40 not touch the lingual-side membranous body 145b. It is preferable that a surface of the lingual-side membranous body 145b on the side of the target model portion 120 be formed at a position corresponding to the lingual-side surface of the actual alveolar bone of the patient. If the model 100 is not that of the lower jaw but the upper jaw of the patient, the lingual-side surface corresponds to the palatine-side surface of the actual alveolar bone of the patient. If the resin material of the model 100 is, for example, transparent or semitransparent, the model 100 is formed in a state in which the fourth three-dimensional image 54 of the implant body and the fifth three-dimensional image 55 of the hole-forming instrument have been removed from the left lower diagram in FIG. 10. The site corresponding to the inferior alveolar bone may be formed in a mesh shape if the positional relationship between the hole-forming instrument 40 and the target model portion 120 can be visually recognized.

The adjunctive instrument 200 includes a guide opening 210 for forming a recessed opening for embedding the implant body 30. If the guide opening 210 and the recessed opening 70 are formed by a rotating drill used as the hole-forming instrument 40, they are formed as circular openings. There may be a case where non-circular openings may be formed, as the guide opening 210 and the recessed opening 70, by a hole-forming instrument 40 that is other than a rotating drill.

In the present embodiment, an example has been described in which two scanners, namely, the first scanner 14 and the second scanner 16, are used. If, for example, the first three-dimensional image 51 and the second three-dimensional image 52 can be acquired by a single scanner, the provision of a plurality of scanners may be eliminated.

The first scanner 14 and the second scanner 16 are owned by, for example, a dentist, and the dentist acquires a three-dimensional image of the jaw of the patient. The formation of the construction by the 3D printer 24 may be performed by a suitable professional. Since a three-dimensional image of the patient may be acquired using, for example, the first scanner 14 and/or the second scanner 16, it is also preferable that the first scanner 14 and/or the second scanner 16 not be included in the system 10. It is also preferable that the 3D printer 24 and/or the milling machine 26 not be included in the system 10.

In the present embodiment, an example has been described in which the adjunctive instrument 200 is created in the system 10, and the adjunctive instrument 200 is used in actual procedures. There may be a case where the adjunctive instrument 200 is not necessarily required through selection of, for example, the hole-forming instrument 40. A treatment plan may be laid in such a manner that the hole-forming instrument 40 including the drill body 42 and the sleeve 46 is placed through the opening 130, as shown in FIG. 18B, instead of placing the adjunctive instrument 200 in the model 100.

### (First Modification)

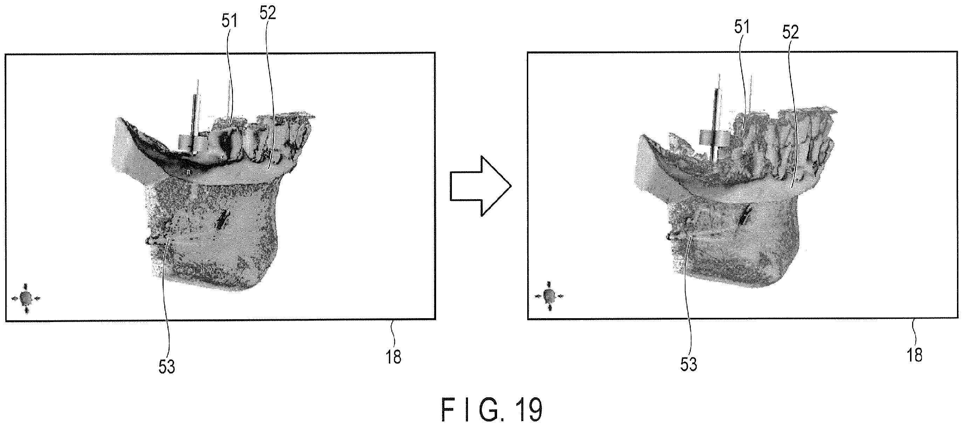

In the system 10 according to the first embodiment, an example has been described in which matching is performed between a CT image (first three-dimensional image) of the lower jaw of the patient and a three-dimensional image (a second three-dimensional image 52) of the lower jaw of the patient, as shown in the left diagram in FIG. 19.

A metal material, for example, may be used as part of the patient's teeth. Due to, for example, noise called artifacts, it is sometimes difficult to obtain a vivid CT image of the patient (first three-dimensional image 51). In this case, it is preferable that, based on the dentist's instruction, the control apparatus 12 allow the first three-dimensional image 51 and the second three-dimensional image 52 to be placed so as to be shifted from each other, while finding points of identity in shape such as the alignment of teeth.

For example, based on the dentist's instruction, the control apparatus 12 superimposes, in the software on the display 18, the second three-dimensional image 52 on the first three-dimensional image 51 on which the third to fifth three-dimensional images 53-55 are superimposed, so as to be shifted from the first three-dimensional image 51, as shown in the right diagram in FIG. 19, beginning at the state shown in the left diagram in FIG. 19. Specifically, the surface of the gums in the second three-dimensional image 52 is, for example, made to match the surface of the alveolar bone, or placed in the alveolar bone in the first three-dimensional image 51. At this time, in the image (see the upper left diagram in FIG. 17) obtained by subtracting the first three-dimensional image 51 from the image obtained by superimposing the second to fifth three-dimensional images 52-55, a distance between the surface of the gums in the second three-dimensional image 52 and the not-to-be-reached targets in the third three-dimensional image 53 is shorter than a distance from the surface of the actual gums of the patient to the not-to-be-reached targets. Thus, the dentist uses, in actual procedures, a hole-forming instrument 40 with a length smaller than the actually usable one, and the fourth three-dimensional image 54 corresponding to the implant body is placed at a depth smaller than an actually embeddable position, relative to the third three-dimensional image 53. Accordingly, the dentist can plan a treatment plan on the safer side.

If there is unreliability in matching between the first three-dimensional image 51 and the second three-dimensional image 52, the second three-dimensional image 52 is caused to be close to the third three-dimensional image 53 within a realistic range. Thus, by planning the implant position in such a manner that the distance between the second three-dimensional image 52 and the third three-dimensional image 53 is equal to or shorter than the actual distance, it is possible to ensure safety. In an actual treatment, based on the dentist's instruction, the control apparatus 12 sets a new second three-dimensional image 52 (first surface data) by shifting characteristic points of the second three-dimensional image 52 (a three-dimensional image of the surface) to the side of an image (target) based on the third three-dimensional image 53 (fourth surface data).

By the way, separating the surface of the gums of the second three-dimensional image 52 from the first three-dimensional image 51 and the third three-dimensional image 53, contrary to the example shown in the right diagram in FIG. 19, could result in laying a treatment plan on a risky side. In the image (see the upper left diagram in FIG. 17) obtained by subtracting the first three-dimensional image 51 from the image obtained by superimposing the second to fifth three-dimensional images 52-55, a distance between the surface of the gums in the second three-dimensional image 52 and the not-to-be-reached targets in the third three-dimensional image 53 is greater than a distance from the surface of the actual gums of the patient to the not-to-be-reached targets. Thus, the dentist may have a mistaken idea that the implant body 30 and the hole-forming instrument 40 may be, for example, placed at a greater depth and set to have a greater length in actual procedures. This may result in the dentist planning a treatment plan on a more risky side.

### (Second Modification)

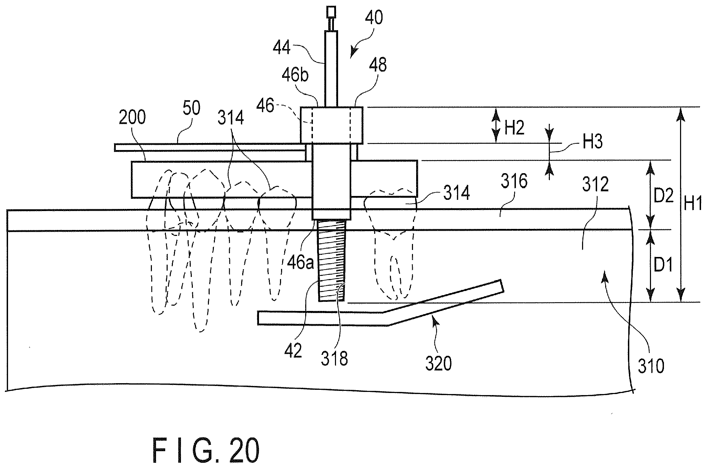

The system 10 according to the second modification of the first embodiment will be described with reference to FIG. 20. Herein, a difference in parameter settings in the case of using a hole-forming instrument 40 different from the hole-forming instrument 40 shown in FIG. 3 will be described.

FIG. 20 shows a first modification of the hole-forming instrument 40 different from that of FIG. 3. The hole-forming instrument 40 shown in FIG. 20 includes a handle 50 in addition to the body 42, the shank 44, the sleeve 46, and the stopper 48. A lower end of the stopper 48 abuts the handle 50. Thus, the position of the distal end of the body 42 of the hole-forming instrument 40 is adjusted by the height of the handle 50. If, for example, the height of the handle 50 increases, a bottom of the recessed opening 318 is distanced from the not-to-be-reached target 320.

As shown in FIG. 20, the dentist selects the hole-forming instrument 40 and the implant body 30 in such a manner that (length D1 of implant body 30)+(offset value D2 during use of adjunctive instrument 200)=(length H1 of drill body 42 from upper end of drill sleeve 46)-(height H2 of stopper 48)-(height H3 of handle 50). Based on the dentist's instruction, the control apparatus 12 sets various setting values in accordance with various hole-forming instruments 40.

That is, the dentist suitably inputs the above-described parameters H1, H2, H3, D1, and D2 using the operation portion 20, using the shape (length and outer diameter), the position, and the angle of the implant body 30 as parameters, and creates an optimum treatment plan while confirming the state relative to the treatment objective site of the patient. The setting or installation of the parameters H1, H2, and H3 may include selecting, by the dentist, an optimum hole-forming instrument 40.

### (Third Modification)

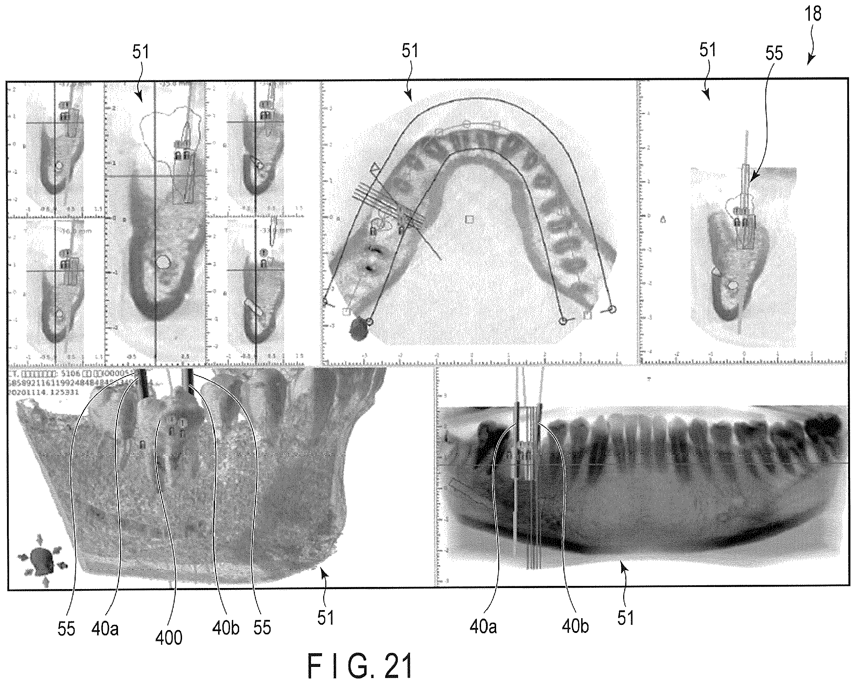

In the first embodiment, an example has been described with respect to the case where, as shown in FIG. 12, the implant body 30 is embedded in the recessed opening 318 formed in the lower jaw. In the case of, for example, forming a recessed opening in which an autologous tooth 400 is embedded as an embedded body, as shown in FIG. 21, instead of embedding the implant body 30 into the recessed opening 318, it is possible to create a treatment plan using the system 10 described in the first embodiment.

The dentist can create a three-dimensional image (second surface data) of the autologous tooth 400, similarly to the creation of the three-dimensional image of the implant body 30. A three-dimensional image of the autologous tooth 400 may be acquired using various pieces of equipment. The three-dimensional image of the autologous tooth 400 may be acquired using, for example, the second scanner 16.

Based on the dentist's instruction, the control apparatus 12 allows the above-described parameters H1, H2, H3, D1, and D2 to be suitably input using the operation portion 20, using the shape, the position, and the angle of the autologous tooth 400 as parameters, and allows an optimum treatment plan to be created while allowing confirmation of the state relative to the treatment objective site of the patient. The setting or installation of the parameters H1, H2, and H3 may include selecting, by the dentist, an optimum hole-forming instrument 40. The autologous tooth 400 has a shape that differs according to the patient, unlike the implant body 30, for which a commercially available one may be used. Thus, in the case of embedding the autologous tooth 400 in the lower jaw, two or three recessed openings may be bored in accordance with the shape of the autologous tooth 400. The adjunctive instrument 200 may also be used in the case of forming such recessed openings. In the case of creating a recessed opening, a plurality of hole-forming instruments 40a and 40b, which are either identical or different, may be used in order.

It is to be noted that, in performing regenerative medicine using, for example, the stem cells of the teeth instead of an autologous tooth, it is possible to perform a treatment of embedding the stem cells of the teeth cultured in vivo or ex vivo into a recessed opening. In this case, based on the dentist's instruction, the control apparatus 12 allows, in creation of a treatment plan in the system 10, the above-described parameters H1, H2, H3, D1, and D2 to be suitably input using the operation portion 20, using the shape, position, and angle of the cell tissues (e.g., an aggregate of cell tissues), which are an embedded body in regenerative medicine, as parameters, and allows an optimum treatment plan to be created, while allowing confirmation of the state relative to the treatment objective site of the patient. The setting or installation of the parameters H1, H2, and H3 may include selecting, by the dentist, an optimum hole-forming instrument 40. In this manner, even in the case of performing regenerative medicine, it is possible for the dentist to create a treatment plan and to create a model for pre-procedure verification of a dental treatment plan using the above-described system 10.

### (Second Embodiment)

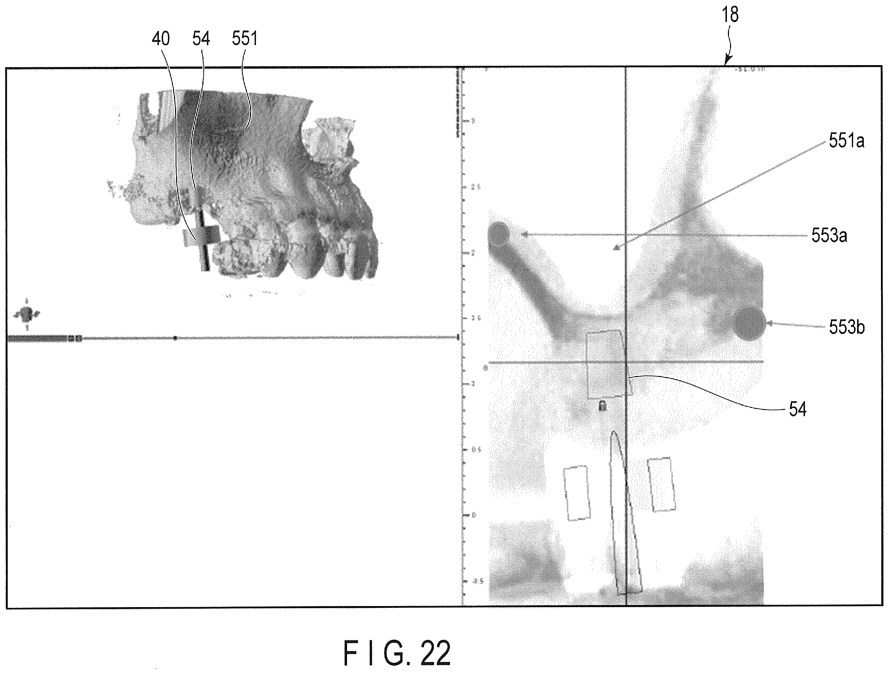

In the second embodiment, with reference to FIGS. 22 to 30, a case will be described where a system 10 is used in an example of performing an implant treatment in which an implant body 30 (see FIG. 2), selected from a multitude, is embedded in the upper jaw of the patient. A description about the matters common to those described in the first embodiment will be suitably omitted.

The first scanner (dental CT scanner) 14 acquires a first three-dimensional image (e.g., DICOM data) 551 of the teeth, the bone, and the inside of the bone of the upper jaw of the patient, and outputs it to the control apparatus 12. The control apparatus 12 causes the storage apparatus 22 to store the first three-dimensional image 551. The second scanner (intraoral scanner) 16 acquires, for example, a second three-dimensional image (e.g., STL data) 552 of, for example, the surface of the teeth and the gums of the upper jaw of the patient, and outputs it to the control apparatus 12. The control apparatus 12 causes the storage apparatus 22 to store the second three-dimensional image 552.

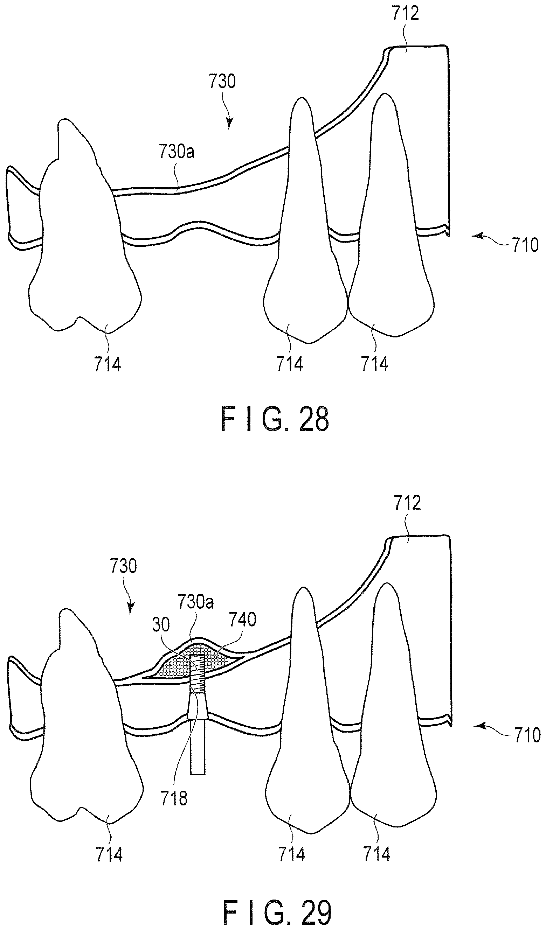

As shown in FIG. 22, the dentist specifies, on the software, the positions of the posterior superior alveolar artery and the greater palatine artery, which are not-to-be-reached targets, and the maxillary sinus floor mucosa (see FIGS. 28 to 30), using the first three-dimensional image 551 showing the superior alveolar bone, the maxillary sinus, the posterior superior alveolar artery, and the greater palatine artery obtained by the first scanner 14 at the time of setting of the recessed opening 70 for embedding the implant body. The dentist marks characteristic points of the not-to-be-reached targets in such a manner that the not-to-be-reached targets are three-dimensionally formed (step S3). The dentist creates not-to-be-reached targets as the third three-dimensional images 553a, 553b, and 553c. It is to be noted that the posterior superior alveolar artery, the greater palatine artery, and the maxillary sinus floor mucosa are adjacent to the superior alveolar bone.

In the right diagram in FIG. 22, a three-dimensional image 553a of the site of the maxillary sinus 551a and the site of the posterior superior alveolar artery, and a three-dimensional image 553b of a site of the greater palatine artery are shown.

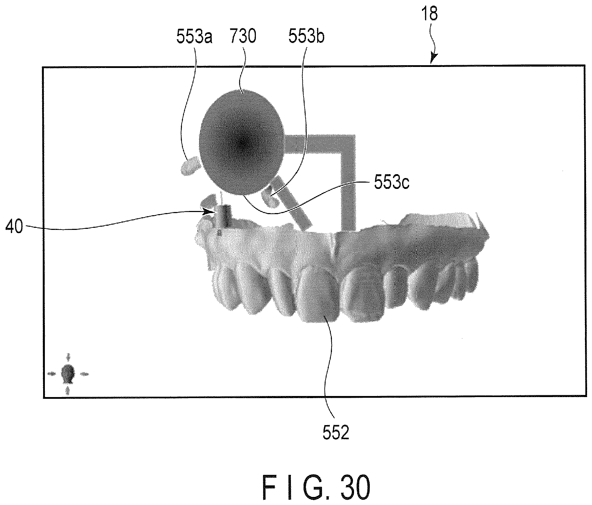

It is to be noted that the three-dimensional image 553c corresponding to the maxillary sinus floor mucosa may be created in a manner similar to a part of an egg shell, or may be formed in a sphere shape, for example, as a whole (see FIG. 30). This is because, in performing advance verification of a treatment plan based on a model, it is a site corresponding to the maxillary sinus floor mucosa that affects the reaching of the distal end of the body 42 of the hole-forming instrument 40, and the remaining site does not affect the reaching of the distal end of the body 42 of the hole-forming instrument 40.

Based on the dentist's instruction, the control apparatus 12 sets or installs, on the software, the length, the outer diameter, the angle, the position, etc. of the implant body that models the implant body 30 at the treatment position, as shown in FIG. 22 (step S4). In this case, based on the dentist's instruction, the control apparatus 12 suitably sets the outer diameter, the position, and the angle of the implant body 30, and a variety of parameters (including selection of the hole-forming instrument 40) described in the first embodiment, and optimizes the treatment plan.

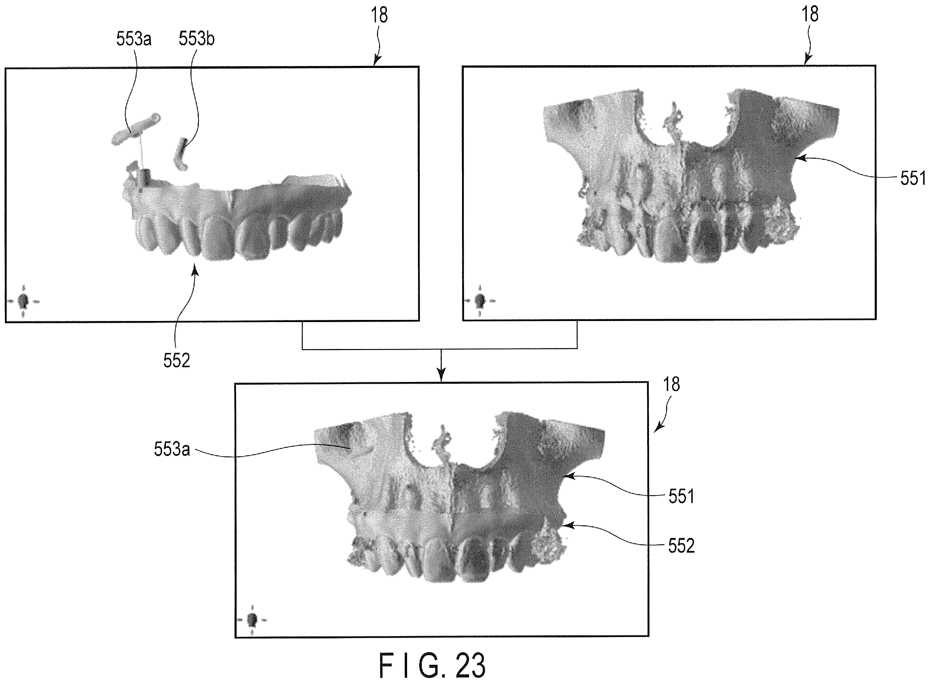

Based on the dentist's instruction, the control apparatus 12 aligns, on the software, coordinate axes of the first to fifth three-dimensional images 551, 552, 553a-553c, 54, and 55, as shown in FIG. 23, and superimposes, on a predetermined coordinate system, the second three-dimensional image 552 on the first three-dimensional image 551, the third three-dimensional images 553a-553c, the fourth three-dimensional image 54 and/or the fifth three-dimensional image 55 (step S5). Thus, the control apparatus 12 clearly shows, to the dentist, the positional relationship between the surface of the teeth and the gums of the upper jaw and the arteries inside the superior alveolar bone on the software.

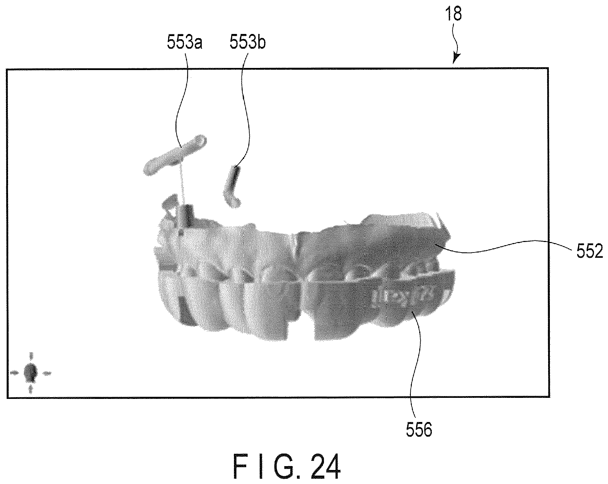

Based on the dentist's instruction, the control apparatus 12 creates an adjunctive instrument (a surgical template) on the software in accordance with the shape of the upper jaw of the patient, as shown in FIG. 24 (step S6). That is, the control apparatus 12 creates an adjunctive instrument as a sixth three-dimensional image 556. The dentist confirms, on the display 18, a state of placement of the sixth three-dimensional image 556, the fourth three-dimensional image 54 or the fifth three-dimensional image 55, and the third three-dimensional images 553a and 553b (step S7). Based on the dentist's instruction, the control apparatus 12 outputs an adjunctive instrument (step S8).

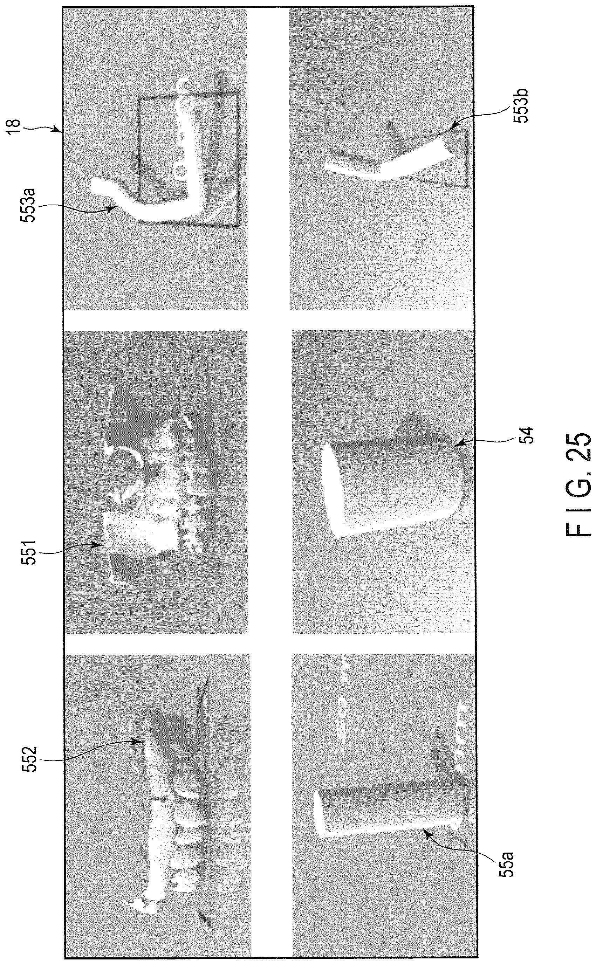

The dentist confirms whether or not there is any problem that should be fixed in the treatment plan on the software. If there is any problem, the problem is fixed. If there is no problem, based on the dentist's instruction, the control apparatus 12 superimposes, on a predetermined coordinate system, the first three-dimensional image 551 showing the teeth and the superior alveolar bone, the second three-dimensional image 552 showing the teeth and the gums, the third three-dimensional images 553a and 553b of the not-to-be-reached targets, the fourth three-dimensional image 54 of the implant body, and the fifth three-dimensional image 55 of the hole-forming instrument 40, as shown in FIG. 25 (step S9). At this time, the control apparatus 12 superimposes, on a predetermined coordinate system, the second three-dimensional image 552, the third three-dimensional images 553a and 553b, the fourth three-dimensional image 54, and the fifth three-dimensional image 55 by means of software same as the software that creates a treatment plan.

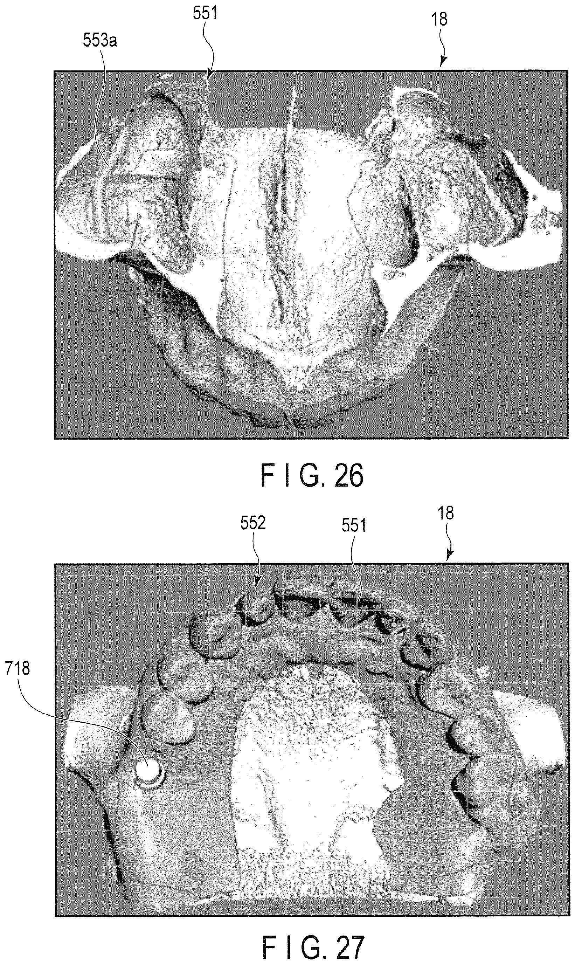

Based on the dentist's instruction, the control apparatus 12 removes, on the software, the fourth three-dimensional image 54 relating to the implant body 30 and the fifth three-dimensional image 55a relating to the sleeve (step S10). That is, the control apparatus 12 creates, on the software, data for the 3D printer 24 (surface data of the model) of the teeth, the gums including a through opening that models a recessed opening for embedding the implant body 30, and the not-to-be-reached targets, as shown in FIGS. 26 and 27.

After the dentist has confirmed the data for the 3D printer 24, based on the dentist's instruction input, the 3D printer 24 constructs, under the control of the control apparatus 12, a model for the patient's treatment site that models the teeth, the gums including a through opening that models the recessed opening for embedding the implant body 30, and the not-to-be-reached targets such as the posterior superior alveolar artery and the greater palatine artery (step S11).

FIG. 28 shows a relationship between the alveolar bone 712, the teeth 714, and the maxillary sinus 730 of the upper jaw 710. FIG. 29 shows the implant body 30 being fixed to the upper jaw 710 using, for example, maxillary sinus floor augmentation such as a sinus lift (socket lift) treatment. There may be a case where the thickness of the superior alveolar bone 712 shown in FIG. 28 is inadequate for embedding the implant body 30 into the superior alveolar bone 712 of the upper jaw 710. At this time, maxillary sinus floor augmentation is performed, in which the maxillary sinus floor mucosa 730a of the maxillary sinus 730 shown in FIG. 29 is lifted using an artificial bone graft material 740. In the case of performing maxillary sinus floor augmentation, the maxillary sinus floor mucosa 730a of the maxillary sinus 730, as well as the posterior superior alveolar bone artery and the greater palatine artery, becomes the not-to-be-reached target of the drill body 42, which is the hole-forming instrument 40.

In the case of performing maxillary sinus floor augmentation, the recessed opening 718 for embedding the implant body 30 is bored in the superior alveolar bone 712. At this time, the recessed opening 718 is bored so as to reach the vicinity of the bottom of the maxillary sinus 730 but to not penetrate the maxillary sinus floor mucosa 730a. The superior alveolar bone 712 is penetrated not by the hole-forming instrument 40 but by an osteotome or the like. The bone graft material 740 is filled between the mucosa 730a of the maxillary sinus 730 and the superior alveolar bone 712. The implant body 30 is embedded in this state. At this time, the bone graft material 740 and the implant body 30 do not break the maxillary sinus floor mucosa 730a.

In this case, as a model for pre-procedure verification of a dental treatment plan, a model that models the teeth, the gums, the posterior superior alveolar bone artery, the greater palatine artery, and the maxillary sinus floor mucosa is constructed, without forming the superior alveolar bone, as shown in FIG. 30, similarly to, for example, the first embodiment in which the inferior alveolar bone is not formed. Such formation of the model allows the dentist to confirm the positional relationship between the distal end of the body 42 of the hole-forming instrument 40 and the maxillary sinus floor mucosa 730a, with the selected hole-forming instrument 40 fitted into the guide opening of the adjunctive instrument and the through opening of the model. That is, the dentist can perform advance verification of the dental treatment plan to verify whether or not a dental treatment can be performed in a safer manner.

It is preferable that the maxillary sinus floor mucosa 730a in the model of the upper jaw, with a site corresponding to the teeth and the gums formed as the model main body, be supported by one or more pillars, as in the model 100 described in the first embodiment.

In the case of using the system 10 in the upper jaw, similarly to the case of using the system 10 in the lower jaw, an advantageous effect similar to that in the lower jaw can be obtained.

As described above, according to the present embodiment, it is possible to provide a pre-procedure verification system 10 of a dental treatment plan, a pre-procedure verification program of a dental treatment plan, a method of manufacturing a model for pre-procedure verification of a dental treatment plan, and a model for pre-procedure verification of a dental treatment plan, which allow the dentist to perform advance verification of the positional relationship between the position of a distal end of a procedural instrument during use of the procedural instrument and the target positions such as the positions of the posterior superior alveolar artery and the greater palatine artery, and the maxillary sinus mucosa of the upper jaw of the patient, prior to, for example, a dental treatment using an actual procedural instrument in performing a treatment of embedding an embedded body such as an implant body.

It is to be noted that, in the system 10 according to the second embodiment, the autologous tooth 400 or cell tissues described in the modification of the first embodiment can be used, instead of the implant body 30.

### (Modification)

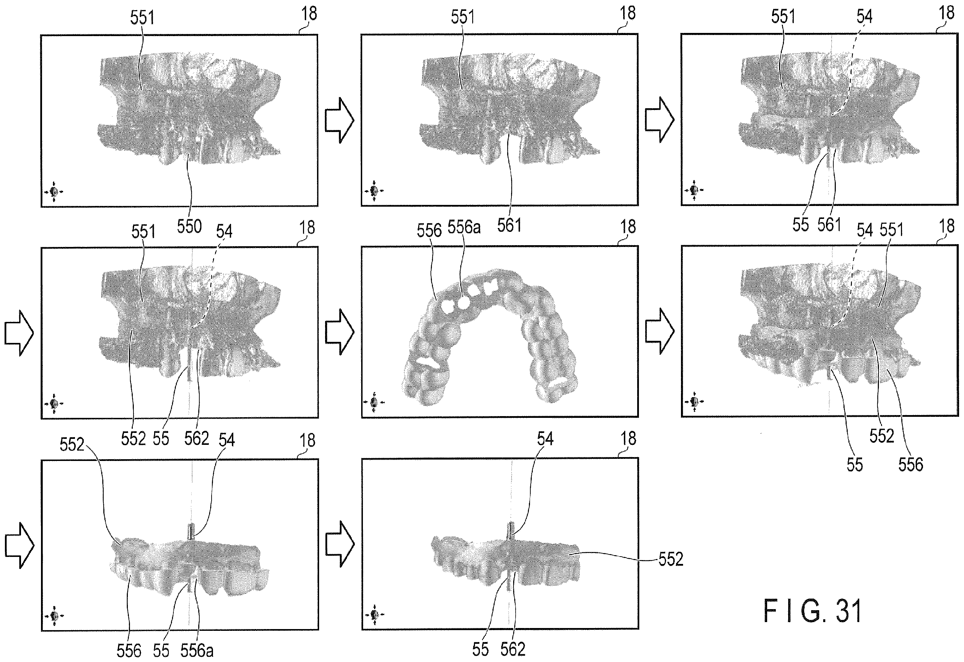

An example of using the upper jaw as shown in FIG. 31 will be described. An operation will be described in which the dentist has judged that an implant treatment is possible (step S2-Yes in the flow shown in FIG. 6). It is to be noted that, in FIG. 31, illustration of an image corresponding to the third three-dimensional image 53 showing the target of the three-dimensional image 100a of the model 100 shown in FIG. 17 is omitted.

For example, there is a case where a currently existing tooth (denoted by the reference numeral 550 in the three-dimensional image 551) is extracted and an implant body 30 is embedded into the position from which the tooth has been extracted. In this case, the tooth 550 to be extracted exists at the treatment objective site of the first three-dimensional image 551 in the upper jaw prior to the treatment. Based on the dentist's instruction, the control apparatus 12 is configured to three-dimensionally cut the tooth 550 to be extracted in the first three-dimensional image 551 and the second three-dimensional image 552. Thus, based on the dentist's instruction, the control apparatus 12 forms a region 561 in the first three-dimensional image 551, and forms a region 562 in the second three-dimensional image 552. The shapes of the regions (spaces) 561 and 562 can be suitably set by the dentist.

The dentist performs the process at step S3 of the flow shown in FIG. 6. Thus, the control apparatus 12 creates, based on the dentist's instruction, three-dimensional images relating to the not-to-be-reached targets (third three-dimensional images 553a, 553b, and 553c (see FIG. 22)). It is to be noted that, in the case of performing the above-described maxillary sinus floor augmentation, the maxillary sinus floor mucosa 730a of the maxillary sinus 730, as well as the posterior superior alveolar bone artery and the greater palatine artery, is the not-to-be-reached target of the drill body 42, which is the hole-forming instrument 40.

Based on the dentist's instruction, the control apparatus 12 performs the process at step S4 in the flow shown in FIG. 6. The control apparatus 12 sets the fourth three-dimensional image 54 corresponding to the implant body in such a manner that a distal end of the implant body 30 is placed at a position that does not reach a specified target, and sets a fifth three-dimensional image 55 corresponding to the hole-forming instrument.

Thereafter, based on the dentist's instruction, the control apparatus 12 performs the processing at steps S5-S11 in the flow shown in FIG. 6.

It is to be noted that the processing at and after step S5 is performed based on three-dimensional images 551 and 552 in which the regions 561 and 562 are formed. Thus, the sixth three-dimensional image 556 corresponding to the surgical guide is formed in such a manner that the tooth image 550 in FIG. 31 does not exist, and the drill hole image 556a is formed in a circular shape that conforms to the shape of the hole-forming instrument 40. It is to be noted that, if the tooth image 550 exists, a deformed space is formed in which the shape of the tooth image 550 is cut out from the drill hole image 556a.

According to the above-described first embodiment and second embodiment including their modifications, the dentist can perform pre-procedure advance verification to verify, prior to performing a treatment of embedding an implant body 30 or an autologous tooth, whether or not there is any problem in the treatment plan, including selection of various treatment instruments, using a model 100 produced using a system 10 for advance verification of a dental treatment, a hole-forming instrument (procedural instrument) 40 to be used in an actual dental treatment, and the implant body 30 or an autologous tooth 400. This prevents the dentist from suspecting whether or not tools used during the procedures (e.g., the hole-forming instrument 40) are adequate for performing a predetermined treatment of the treatment objective site of the patient in accordance with the treatment plan, thus taking a long time for the treatment and putting a burden on the patient. Thus, by using the system 10 and the model 100 according to the above-described first embodiment and second embodiment including their modifications and the hole-forming instrument 40 set in the treatment plan, it is possible to perform a less-invasive dental treatment on the patient.

Also, by allowing the dentist, not the professional, to perform the operation of creating a treatment plan using the system 10 until a three-dimensional image of the model 100 is created, while allowing the dentist who actually performs the treatment to perform confirmation, it is possible to perform a treatment of embedding, for example, the implant body 30 or an autologous tooth in a safer manner.

In the case where the dentist owns the 3D printer 24 or the milling machine 26, it is possible to omit the time of transfer of the three-dimensional image and the time of conveyance of the model 100, thus reducing the time taken for a series of operations from the creation of the treatment plan to the advance verification of the dental treatment using the model 100. In the case of fixing a treatment plan, it is possible to reduce the time taken for a series of operations from the correction of the treatment plan to the advance verification of the dental treatment using the model 100.

## Claims

1. A model (100) for pre-procedure verification of a dental treatment plan, comprising:
a model main body (110) formed based on a jaw of a patient in a size and a shape identical to at least a part of a treatment objective site of the jaw of the patient and configured to model the at least a part of the treatment objective site;
an opening defining portion (130) provided in the model main body (110) and configured to define an opening corresponding to a recessed opening (70, 318, 718) for embedding an embedded body in the treatment objective site; and
a target model portion (120) configured to model a target adjacent to or embedded in an alveolar bone (312, 712) of the treatment objective site,
the target model portion (120) allowing a distal end of a procedural instrument used for a treatment to be out of contact with the target model portion (120) under a condition that the procedural instrument passes through the opening defining portion (130) in a positional relationship of the target model portion (120) identical to a positional relationship of the target relative to the treatment objective site, or
the target model portion (120) allowing the distal end of the procedural instrument to be in contact with the target model portion (120) under a condition that the procedural instrument passes through the opening defining portion (130) in a positional relationship of the target model portion (120) closer than the positional relationship of the target relative to the treatment objective site,
wherein:
a space (145) is formed between the opening defining portion (130) and the target model portion (120) to allow visual recognition of a range present between the opening defining portion (130) and the target model portion (120), the range including a reachable range of the distal end of the procedural instrument from the opening defining portion (130) toward the target model portion (120) and a reachable range of the distal end of the procedural instrument from the opening defining portion (130) in a teeth alignment direction and a direction intersecting the teeth alignment direction.

2. The model (100) according to claim 1, wherein:
the target model portion (120) is supported on the model main body (110) with a main pillar (160), and
the main pillar (160) is formed as a part of a frame that defines the space (145).

3. The model (100) according to claim 1 or 2,
wherein:
The model main body (110) comprises a lingual-side or palatine-side wall that models a surface of the alveolar bone (312, 712).

4. The model (100) according to any one of claims 1 to 3, comprising:
a base portion (140) which allows the target model portion (120) to be placed between the base portion (140) and the model main body (110); and
at least one of:
a pillar or pillars (150) connecting the model main body (110) and the base portion (140) and connecting the model main body (110) and the target model portion (120) ;
a pillar or pillars (150) connecting the model main body (110) and the base portion (140) and connecting the base portion (140) and the target model portion (120); and
a pillar or pillars (150) connecting the model main body (110) and the target model portion (120) and connecting the base portion (140) and the target model portion (120).

5. A pre-procedure verification instrument (260) of a dental treatment plan, comprising:
the model (100) according to any one of claims 1 to 4; and
an adjunctive instrument (200) configured to fit into or engage with the model (100) and to guide the procedural instrument to form the opening defining portion (130) at a desired position.

6. A method of manufacturing a model (100) for pre-procedure verification of a dental treatment plan, comprising:
causing, based on an instruction by a dental treatment planner, a computer (12) to specify, in a three-dimensional stereo image of a jaw of a patient, a treatment objective site of the patient and a target that is to be out of contact or in contact with a distal end of a procedural instrument used for a treatment on the treatment objective site;
causing, based on the instruction by the dental treatment planner, the computer (12) to set, in the three-dimensional stereo image of the jaw of the patient, an opening that models a recessed opening (70, 318, 718) for embedding an embedded body to be embedded in the treatment objective site by setting or installing at least one of a parameter relating to the embedded body and a parameter relating to the procedural instrument; and
causing, based on the instruction by the dental treatment planner, the computer (12) to create a three-dimensional stereo image based on fifth surface data (100a) of the model (100), the fifth surface data (100a) being obtained by subtracting at least one of second surface data (54) of a surface of the embedded body and third surface data (55) of a surface of the procedural instrument from first surface data (52, 552) of the treatment objective site and a peripheral site thereof, the fifth surface data (100a) indicating a positional relationship between the treatment objective site including the opening and fourth surface data (53, 553a, 553b, 553c) of the target,
wherein the causing the computer (12) to specify the target includes specifying an image of a main pillar (160) joined to an image based on the first surface data (52, 552),
the manufacturing method comprising:
forming the model (100) based on the fifth surface data (100a), the model (100) comprising:
a model main body (110) in a size and a shape identical to at least a part of the treatment objective site and configured to model the at least a part of the treatment objective site;
an opening defining portion (130) provided in the model main body (110) and configured to define the opening; and
a target model portion (120) configured to model the target, and to allow the distal end of the procedural instrument to be out of contact with the target model portion (120) under a condition that the procedural instrument passes through the opening defining portion (130) in a positional relationship of the target model portion (120) identical to a positional relationship of the target relative to the treatment objective site, or to allow the distal end of the procedural instrument to be in contact with the target model portion (120) under a condition that the procedural instrument passes through the opening defining portion (130) in a positional relationship of the target model portion (120) closer than the positional relationship of the target relative to the treatment objective site,
wherein the forming the model (100) includes forming a space (145) which is formed between the opening defining portion (130) and the target model portion (120) to allow visual recognition of a range present between the opening defining portion (130) and the target model portion (120), the range including a reachable range of the distal end of the procedural instrument from the opening defining portion (130) toward the target model portion (120) and a reachable range of the distal end of the procedural instrument from the opening defining portion (130) in a teeth alignment direction and a direction intersecting the teeth alignment direction.

7. A method of manufacturing a model (100) for pre-procedure verification of a dental treatment plan, comprising:
causing, based on an instruction by a dental treatment planner, a computer (12) to specify, in a three-dimensional stereo image of a jaw of a patient, a treatment objective site of the patient and a target that is to be out of contact or in contact with a distal end of a procedural instrument used for a treatment on the treatment objective site;
causing, based on the instruction by the dental treatment planner, the computer (12) to set, in the three-dimensional stereo image of the jaw of the patient, an opening that models a recessed opening (70, 318, 718) for embedding an embedded body to be embedded in the treatment objective site by setting or installing at least one of a parameter relating to the embedded body and a parameter relating to the procedural instrument; and
causing, based on the instruction by the dental treatment planner, the computer (12) to create a three-dimensional stereo image of an adjunctive instrument (200) based on
at least one of the parameter relating to the embedded body and the parameter relating to the procedural instrument and
the first surface data (52, 552),
the adjunctive instrument (200) being configured to fit into a periphery of the treatment objective site for use and to guide the procedural instrument to form the recessed opening (70, 318, 718) at a desired position,
causing, based on the instruction by the dental treatment planner, the computer (12) to create a three-dimensional stereo image based on fifth surface data (100a) of the model (100), the fifth surface (100a) data being obtained by subtracting at least one of second surface data (54) of a surface of the embedded body and third surface data (55) of a surface of the procedural instrument from first surface data (52, 552) of the treatment objective site and a peripheral site thereof, the fifth surface data (100a) indicating a positional relationship between the treatment objective site including the opening and fourth surface data (53, 553a, 553b, 553c) of the target,
wherein the causing the computer (12) to specify the target includes specifying an image of a main pillar (160) joined to an image based on the first surface data (52, 552),
wherein:
the creating of the three-dimensional stereo image based on the fifth surface data (100a) includes outputting, by the computer (12), the three-dimensional stereo image of the adjunctive instrument (200) for output of the adjunctive instrument (200), and
the creating of the three-dimensional stereo image of the adjunctive instrument (200) is performed prior to outputting, by the computer (12), of the three-dimensional stereo image based on the fifth surface data (100a) for output of the model (100).

8. The manufacturing method according to claim 6 or 7, comprising:
setting the first surface data (52, 552) by matching characteristic points of a three-dimensional image of the surface of the treatment objective site and the peripheral site thereof with characteristic points of the three-dimensional stereo image of the jaw of the patient, or
setting the first surface data (52, 552) by shifting the characteristic points of the three-dimensional image of the surface to a side of an image based on the fourth surface data (53, 553a, 553b, 553c) of the target, with respect to the characteristic points of the three-dimensional stereo image of the jaw of the patient.

9. A pre-procedure verification system (10) of a dental treatment plan, comprising:
a control apparatus (12); and
an instruction input portion (20) which is controlled by the control apparatus (12) and configured to receive input of instructions for the control apparatus (12), the instructions including:
a processing instruction to specify, in a three-dimensional stereo image of a jaw of a patient, a treatment objective site of the patient and a target that is to be out of contact or in contact with a distal end of a procedural instrument used for a treatment on the treatment objective site;
a processing instruction to set, in the three-dimensional stereo image of the jaw of the patient, an opening that models a recessed opening (70, 318, 718) for embedding an embedded body to be embedded in the treatment objective site by setting or installing at least one of a parameter relating to the embedded body and a parameter relating to the procedural instrument; and
a processing instruction to create a three-dimensional stereo image based on fifth surface data (100a) of a model (100) for pre-procedure verification of the dental treatment plan, the fifth surface data (100a) being obtained by subtracting at least one of second surface data (54) of a surface of the embedded body and third surface data (55) of a surface of the procedural instrument from first surface data (52, 552) of the treatment objective site and a peripheral site thereof, the fifth surface data (100a) indicating a positional relationship between the treatment objective site including the opening and fourth surface data (53, 553a, 553b, 553c) of the target,
wherein:
the processing instruction to specify the target includes an instruction to specify an image of a main pillar (160) joined to an image based on the first surface data (52, 552), and
the control apparatus (12) is configured to perform processing based on the processing instructions, and is configured to output the three-dimensional stereo image based on the fifth surface data (100a) for output of the model (100),
wherein:
the instruction input portion (20) is configured to receive input of a processing instruction to create a three-dimensional stereo image of an adjunctive instrument (200) based on at least one of the parameter relating to the embedded body and the parameter relating to the procedural instrument and the first surface data (52, 552), the adjunctive instrument (200) being configured to fit into a periphery of the treatment objective site for use and to guide the procedural instrument to form the recessed opening (70, 318, 718) at a desired position, and
the control apparatus (12) is configured to perform processing based on the processing instructions, and is configured to output the three-dimensional stereo image based on the fifth surface data (100a) for output of the model (100) and the three-dimensional stereo image of the adjunctive instrument (200) for output of the adjunctive instrument (200), and creation of the three-dimensional stereo image of the adjunctive instrument (200) based on the processing instructions is performed prior to the outputting of the three-dimensional stereo image based on the fifth surface data (100a).

10. The pre-procedure verification system (10) according to claim 9, wherein upon insertion of the procedural instrument into the opening in the three-dimensional stereo image of the patient, the parameter relating to the procedural instrument is set or installed as the third surface data (55) so that the procedural instrument is separated from or brought in contact with the target.

11. The pre-procedure verification system (10) according to claim 9 or 10, wherein a positional relationship between the target and the opening in the fourth surface data (53, 553a, 553b, 553c) of the target is closer than a positional relationship between an actual target of the jaw of the patient and the recessed opening (70, 318, 718).

12. The pre-procedure verification system (10) according to claim 11, wherein the first surface data (52, 552) is set by shifting characteristic points of a three-dimensional image of the surface to a side of an image based on the fourth surface data (53, 553a, 553b, 553c) of the target, with respect to characteristic points of the three-dimensional stereo image of the jaw of the patient.

13. The pre-procedure verification system (10) according to claim 9 or 10, further comprising:
a three-dimensional printer (24) configured to output the model (100) based on the three-dimensional stereo image based on the fifth surface data (100a).

14. A pre-procedure verification program of a dental treatment plan, the program causing a computer (12) to execute:
a first process of specifying, in a three-dimensional stereo image of a jaw of a patient, a treatment objective site of the patient and a target that is to be out of contact or in contact with a distal end of a procedural instrument used for a treatment on the treatment objective site;
a second process of setting, in the three-dimensional stereo image of the jaw of the patient, an opening that models a recessed opening (70, 318, 718) for embedding an embedded body to be embedded in the treatment objective site by setting or installing at least one of a parameter relating to the embedded body and a parameter relating to the procedural instrument;
a third process of creating a three-dimensional stereo image based on fifth surface data (100a) of a model (100) for pre-procedure verification of the dental treatment plan, the fifth surface data (100a) being obtained by subtracting at least one of second surface data (54) of a surface of the embedded body and third surface data (55) of a surface of the procedural instrument from first surface data (52, 552) of the treatment objective site and a peripheral site thereof, the fifth surface data (100a) indicating a positional relationship between the treatment objective site including the opening and fourth surface data (53, 553a, 553b, 553c) of the target; and
a fourth process of outputting the three-dimensional stereo image based on the fifth surface data (100a) of the model (100),
wherein:
the specifying the target includes specifying an image of a main pillar (160) joined to an image based on the first surface data (52, 552),
the program causing the computer (12) to execute:
forming the model (100) based on the fifth surface data (100a), the model (100) comprising:
a model main body (110) in a size and a shape identical to at least a part of the treatment objective site and configured to model the at least a part of the treatment objective site;
an opening defining portion (130) provided in the model main body (110) and configured to define the opening; and
a target model portion (120) configured to model the target, and to allow the distal end of the procedural instrument to be out of contact with the target model portion (120) under a condition that the procedural instrument passes through the opening defining portion (130) in a positional relationship of the target model portion (120) identical to a positional relationship of the target relative to the treatment objective site, or to allow the distal end of the procedural instrument to be in contact with the target model portion (120) under a condition that the procedural instrument passes through the opening defining portion (130) in a positional relationship of the target model portion (120) closer than the positional relationship of the target relative to the treatment objective site,
wherein the forming the model (100) includes forming a space (145) which is formed between the opening defining portion (130) and the target model portion (120) to allow visual recognition of a range present between the opening defining portion (130) and the target model portion (120), the range including a reachable range of the distal end of the procedural instrument from the opening defining portion (130) toward the target model portion (120) and a reachable range of the distal end of the procedural instrument from the opening defining portion (130) in a teeth alignment direction and a direction intersecting the teeth alignment direction.

## Patentansprüche

1. Modell (100) zur Verifizierung eines zahnärztlichen Behandlungsplans vor dem Eingriff, umfassend:
einen Modellhauptkörper (110), der auf der Grundlage eines Kiefers eines Patienten in einer Größe und einer Form gebildet ist, die mit mindestens einem Teil einer Behandlungszielstelle des Kiefers des Patienten identisch ist, und der so konfiguriert ist, dass er den mindestens einen Teil der Behandlungszielstelle modelliert;
einen eine Öffnung definierenden Abschnitt (130), der in dem Modellhauptkörper (110) vorgesehen und so konfiguriert ist, dass er eine Öffnung definiert, die einer ausgesparten Öffnung (70, 318, 718) zum Einbetten eines eingebetteten Körpers in die Behandlungszielstelle entspricht; und
einen Zielmodellabschnitt (120), der so konfiguriert ist, dass er ein Ziel modelliert, das an einen Alveolarknochen (312, 712) der Behandlungszielstelle angrenzt oder darin eingebettet ist,
der Zielmodellabschnitt (120) es ermöglicht, dass ein distales Ende eines für eine Behandlung verwendeten Verfahrensinstruments nicht mit dem Zielmodellabschnitt (120) in Kontakt ist, wenn das Verfahrensinstrument durch den die Öffnung definierenden Abschnitt (130) in einer Positionsbeziehung des Zielmodellabschnitts (120) hindurchgeht, die identisch mit einer Positionsbeziehung des Ziels in Bezug auf die Behandlungszielstelle ist, oder
der Zielmodellabschnitt (120) es dem distalen Ende des Verfahrensinstruments ermöglicht, mit dem Zielmodellabschnitt (120) unter der Bedingung in Kontakt zu sein, dass das Verfahrensinstrument durch den die Öffnung definierenden Abschnitt (130) in einer Positionsbeziehung des Zielmodellabschnitts (120) verläuft, die näher als die Positionsbeziehung des Ziels relativ zum Behandlungszielort ist,
wobei:
ein Zwischenraum (145) zwischen dem die Öffnung definierenden Abschnitt (130) und dem Zielmodellabschnitt (120) gebildet wird, um eine visuelle Erkennung eines zwischen der Öffnung und dem Zielmodellabschnitt vorhandenen Bereichs zu ermöglichen definierenden Abschnitt (130) und dem Zielmodellabschnitt (120), wobei der Bereich einen erreichbaren Bereich des distalen Endes des Verfahrensinstruments von dem die Öffnung definierenden Abschnitt umfasst (130) in Richtung des Zielmodellabschnitts (120) und eines erreichbaren Bereichs des distalen Endes des Verfahrensinstruments von dem die Öffnung definierenden Teil (130) in einer Zahnausrichtungsrichtung und einer die Zahnausrichtungsrichtung schneidenden Richtung.

2. Das Modell (100) nach Anspruch 1, wobei:
der Zielmodellabschnitt (120) auf dem Modellhauptkörper (110) mit einer Hauptsäule (160) getragen wird, und der Hauptpfeiler (160) ist Teil eines Rahmens, der den Raum (145) definiert.

3. Das Modell (100) nach Anspruch 1 oder 2, wobei:
der Modellhauptkörper (110) umfasst eine linguale oder palatinseitige Wand, die eine Oberfläche des Alveolarknochens (312, 712) modelliert.

4. Das Modell (100) nach einem der Ansprüche 1 bis 3, umfassend:
einen Basisteil (140), der es ermöglicht, den Zielmodellabschnitt (120) zwischen dem Basisteil (140) und dem Modellhauptkörper (110) zu platzieren; und mindestens eines von:
eine oder mehrere Säulen (150), die den Modellhauptkörper (110) und den Basisabschnitt (140) sowie den Modellhauptkörper (110) und den Zielmodellabschnitt (120) verbinden;
eine oder mehrere Säulen (150), die den Modellhauptkörper (110) und den Basisabschnitt (140) sowie den Basisabschnitt (140) und den Zielmodellabschnitt (120) verbinden;
und eine Säule oder Säulen (150), die den Modellhauptkörper (110) und den Zielmodellabschnitt (120) verbinden und den Basisabschnitt (140) und den Zielmodellabschnitt (120) verbinden.

5. Instrument (260) zur Überprüfung eines zahnärztlichen Behandlungsplans vor dem Eingriff, umfassend:
das Modell (100) nach einem der Ansprüche 1 bis 4; und ein Zusatzinstrument (200), das so konfiguriert ist, dass es in das Modell (100) passt oder damit in Eingriff kommt und das Verfahrensinstrument führt, um den die Öffnung definierenden Abschnitt (130) an einer gewünschten Position zu bilden.

6. Verfahren zur Herstellung eines Modells (100) zur Verifizierung eines zahnärztlichen Behandlungsplans vor dem Eingriff, umfassend: Veranlassung eines Computers (12) auf der Grundlage einer Anweisung eines zahnärztlichen Behandlungsplaners, in einem dreidimensionalen Stereobild eines Kiefers eines Patienten eine Behandlungszielstelle des Patienten und ein Ziel zu spezifizieren, das mit einem distalen Ende eines Verfahrensinstruments, das für eine Behandlung an der Behandlungszielstelle verwendet wird, außer Kontakt oder in Kontakt sein soll; Bewirken, dass der Computer (12) auf der Grundlage der Anweisung des Zahnbehandlungsplaners in dem dreidimensionalen Stereobild des Kiefers des Patienten eine Öffnung einstellt, die eine vertiefte Öffnung (70, 318, 718) zum Einbetten eines eingebetteten Körpers modelliert, der in die Behandlungszielstelle eingebettet werden soll, indem ein Parameter, der sich auf den eingebetteten Körper bezieht, und/oder ein Parameter, der sich auf das Verfahrensinstrument bezieht, eingestellt oder installiert wird; und
Veranlassen des Computers (12), basierend auf der Anweisung des Zahnbehandlungsplaners, ein dreidimensionales Stereobild zu erzeugen, das auf fünften Oberflächendaten (100a) des Modells (100) basiert, wobei die fünften Oberflächendaten (100a) durch Subtraktion von mindestens einem der zweiten Oberflächendaten (54) einer Oberfläche des eingebetteten Körpers und dritten Oberflächendaten (55) einer Oberfläche des Verfahrensinstruments aus ersten Oberflächendaten (52, 552) der Behandlungszielstelle und einer peripheren Stelle davon erhalten werden, wobei die fünften Oberflächendaten (100a) eine Positionsbeziehung zwischen der Behandlungsziel, einschließlich der Daten der ersten und vierten Oberfläche (53, 553a, 553b, 553c) des Ziels,
wobei das Veranlassen des Computers (12), das Ziel zu spezifizieren, das Spezifizieren eines Bildes eines Hauptpfeilers (160), der mit einem Bild verbunden ist, das auf den ersten Oberflächendaten (52, 552) basiert, beinhaltet, das Herstellungsverfahren umfasst:
Bilden des Modells (100) auf der Grundlage der fünften Oberflächendaten (100a), wobei das Modell (100) umfasst:
einen Modellhauptkörper (110) in einer Größe und Form, die mit mindestens einem Teil des Behandlungsziels identisch ist, und der so konfiguriert ist, dass er den mindestens einen Teil des Behandlungsziels modelliert;
einen eine Öffnung definierenden Abschnitt (130), der in dem Modellhauptkörper (110) vorgesehen und so konfiguriert ist, dass er die Öffnung definiert; und
einen Zielmodellabschnitt (120), der so konfiguriert ist, dass er das Ziel modelliert und es dem distalen Ende des Verfahrensinstruments ermöglicht, unter einer Bedingung, dass das Verfahrensinstrument durch den die Öffnung definierenden Abschnitt hindurchgeht, nicht in Kontakt mit dem Zielmodellabschnitt (120) zu sein (130) in einer Positionsbeziehung des Zielmodellabschnitts (120), die identisch ist mit einer Positionsbeziehung des Ziels relativ zu der Behandlungszielstelle, oder um zu ermöglichen, dass das distale Ende des Verfahrensinstruments in Kontakt mit dem Zielmodellabschnitt (120) unter einer Bedingung ist, dass das Verfahrensinstrument durch den die Öffnung definierenden Abschnitt (130) in einer Positionsbeziehung des Zielmodellabschnitts (120) näher als die Positionsbeziehung des Ziels relativ zu der Behandlungszielstelle hindurchgeht,
wobei das Bilden des Modells (100) das Bilden eines Raums (145) einschließt, der zwischen dem die Öffnung definierenden Abschnitt (130) und dem Zielmodellabschnitt (120) gebildet wird, um eine visuelle Erkennung eines zwischen dem die Öffnung definierenden Abschnitt (130) und dem Zielmodellabschnitt (120) vorhandenen Bereichs zu ermöglichen, der Bereich umfasst einen erreichbaren Bereich des distalen Endes des Verfahrensinstruments von dem die Öffnung definierenden Teil (130) in Richtung des Zielmodellabschnitts (120) und eines erreichbaren Bereichs des distalen Endes des Verfahrensinstruments von dem die Öffnung definierenden Teil (130) in einer Zahnausrichtungsrichtung und einer die Zahnausrichtungsrichtung schneidenden Richtung.

7. Verfahren zur Herstellung eines Modells (100) zur Verifizierung eines zahnärztlichen Behandlungsplans vor dem Eingriff, umfassend:
Veranlassung eines Computers (12) auf der Grundlage einer Anweisung eines zahnärztlichen Behandlungsplaners, in einem dreidimensionalen Stereobild eines Kiefers eines Patienten eine Behandlungszielstelle des Patienten und ein Ziel zu spezifizieren, das mit einem distalen Ende eines Verfahrensinstruments, das für eine Behandlung an der Behandlungszielstelle verwendet wird, außer Kontakt oder in Kontakt sein soll;
Bewirken, dass der Computer (12) auf der Grundlage der Anweisung des Zahnbehandlungsplaners in dem dreidimensionalen Stereobild des Kiefers des Patienten eine Öffnung einstellt, die eine vertiefte Öffnung (70, 318, 718) zum Einbetten eines eingebetteten Körpers modelliert, der in die Behandlungszielstelle eingebettet werden soll, indem ein Parameter, der sich auf den eingebetteten Körper bezieht, und/oder ein Parameter, der sich auf das Verfahrensinstrument bezieht, eingestellt oder installiert wird; und
Veranlassung des Computers (12), basierend auf der Anweisung des zahnärztlichen Behandlungsplaners, ein dreidimensionales Stereobild eines Zusatzinstruments (200) zu erzeugen, das auf mindestens einen der Parameter, die sich auf den eingebetteten Körper beziehen, und den Parameter, der sich auf das verfahrenstechnische Instrument bezieht, und
die ersten Oberflächendaten (52, 552), das Zusatzinstrument (200) so konfiguriert ist, dass es zur Verwendung in eine Peripherie der Behandlungszielstelle passt und das Verfahrensinstrument führt, um die Aussparung (70, 318, 718) an einer gewünschten Position,
Verwendung des Computers (12), basierend auf der Meinung des zahnärztlichen Behandlungsplaners, ein dreidimensionales Stereobild auf der Grundlage von Daten der fünften Oberfläche (100a) des Modells (100) zu erzeugen, wobei die Daten der fünften Oberfläche (100a) durch Subtraktion von mindestens einem der Daten der zweiten Oberfläche (54) einer Oberfläche des eingebetteten Körpers und der dritten Oberflächendaten (55) einer Oberfläche des Verfahrensinstruments aus ersten Oberflächendaten (52, 552) der Behandlungszielstelle und einer peripheren Stelle davon erhalten werden,
wobei die fünften Oberflächendaten (100a) eine Positionsbeziehung zwischen der Behandlungszielstelle einschließlich der Öffnung und der vierten Oberflächendaten (53, 553a, 553b, 553c) des Ziels,
wobei das Veranlassen des Computers (12), das Ziel zu spezifizieren, das Spezifizieren eines Bildes eines Hauptpfeilers (160) umfasst, der mit einem Bild verbunden ist, das auf den ersten Oberflächendaten (52, 552) basiert,
wobei:
das Erzeugen des dreidimensionalen Stereobildes auf der Grundlage der fünften Oberflächendaten (100a) das Ausgeben des dreidimensionalen Stereobildes des Zusatzinstruments (200) durch den Computer (12) zur Ausgabe des Zusatzinstruments (200) umfasst, und
die Erzeugung des dreidimensionalen Stereobildes des Zusatzinstruments (200) vor der Ausgabe des dreidimensionalen Stereobildes durch den Computer (12) auf der Grundlage der fünften Oberflächendaten (100a) für die Ausgabe des Modells (100) durchgeführt wird.

8. Herstellungsverfahren nach Anspruch 6 oder 7 umfassend:
Einstellung der ersten Oberflächendaten (52, 552) durch Abgleich charakteristische Punkte eines dreidimensionalen Bildes der Oberfläche des Behandlungsziels und seiner Peripherie mit charakteristischen Punkten des dreidimensionalen Stereobildes des Kiefers des Patienten, oder
Einstellen der ersten Oberflächendaten (52, 552) durch Verschieben der charakteristischen Punkte des dreidimensionalen Bildes der Oberfläche auf eine Seite eines Bildes, das auf den vierten Oberflächendaten (53, 553a, 553b, 553c) des Ziels basiert, in Bezug auf die charakteristischen Punkte des dreidimensionalen Stereobildes des Kiefers des Patienten.

9. System (10) zur Überprüfung eines zahnärztlichen Behandlungsplans vor dem Eingriff, das Folgendes umfasst:
ein Steuergerät (12); und
einen Befehlseingabeabschnitt (20), der von der Steuervorrichtung (12) gesteuert wird und so konfiguriert ist, dass er die Eingabe von Befehlen für die Steuervorrichtung (12) empfängt, wobei die Befehle umfassen:
eine Verarbeitungsanweisung, um in einem dreidimensionalen Stereobild eines Kiefers eines Patienten eine Behandlungszielstelle des Patienten und ein Ziel zu spezifizieren, das mit einem distalen Ende eines für eine Behandlung an der Behandlungszielstelle verwendeten Verfahrensinstruments ohne Kontakt oder in Kontakt sein soll;
eine Verarbeitungsanweisung, um in dem dreidimensionalen Stereobild des Kiefers des Patienten eine Öffnung einzustellen, die eine vertiefte Öffnung (70, 318, 718) zum Einbetten eines eingebetteten Körpers modelliert, der in die Behandlungszielstelle eingebettet werden soll, indem mindestens einer von einem Parameter, der sich auf den eingebetteten Körper bezieht, und einem Parameter, der sich auf das Verfahrensinstrument bezieht, eingestellt oder installiert wird; und eine Verarbeitungsanweisung zur Erzeugung eines dreidimensionalen Stereobildes auf der Grundlage von fünften Oberflächendaten (100a) eines Modells (100) zur Überprüfung des zahnärztlichen Behandlungsplans vor dem Eingriff, wobei die fünften Oberflächendaten (100a) durch Subtraktion von mindestens einem der zweiten Oberflächendaten (54) einer Oberfläche des eingebetteten Körpers und einer dritten Oberfläche Daten (55) einer Oberfläche des Verfahrensinstruments aus ersten Oberflächendaten (52, 552) der Behandlungszielstelle und einer peripheren Stelle davon erhalten werden, wobei die fünften Oberflächendaten (100a) eine Positionsbeziehung zwischen der Behandlungszielstelle einschließlich der Öffnung und vierten Oberflächendaten (53, 553a, 553b, 553c) des Ziels angeben, wobei:
die Verarbeitungsanweisung zum Spezifizieren des Ziels eine Anweisung zum Spezifizieren eines Bildes eines Hauptpfeilers (160) enthält, der mit einem auf den ersten Oberflächendaten (52, 552) basierenden Bild verbunden ist, und
die Steuervorrichtung (12) so konfiguriert ist, dass sie die Verarbeitung auf der Grundlage der Verarbeitungsanweisungen durchführt, und so konfiguriert ist, dass sie das dreidimensionale Stereobild auf der Grundlage der fünften Oberflächendaten (100a) für die Ausgabe des Modells, (100) ausgibt, wobei:
der Befehlseingabeabschnitt (20) so konfiguriert ist, dass er die Eingabe eines Verarbeitungsbefehls zur Erzeugung eines dreidimensionalen Stereobildes eines Zusatzinstruments empfängt (200) auf der Grundlage mindestens eines der Parameter, die sich auf den eingebetteten Körper beziehen, und der Parameter, die sich auf das Verfahrensinstrument und die ersten Oberflächendaten (52, 552) beziehen, wobei das Zusatzinstrument (200) so konfiguriert ist, dass es zur Verwendung in eine Peripherie der Behandlungszielstelle passt und das Verfahrensinstrument führt, um die ausgesparte Öffnung (70, 318, 718) an einer gewünschten Position zu bilden, und die Steuervorrichtung (12) so konfiguriert ist, dass sie eine Verarbeitung auf der Grundlage der Verarbeitungsanweisungen durchführt, und so konfiguriert ist, dass sie das dreidimensionale Stereobild auf der Grundlage der fünften Oberflächendaten (100a) für die Ausgabe des Modells (100) und das dreidimensionale Stereobild des Zusatzinstruments (200) für die Ausgabe des Zusatzinstruments (200) ausgibt, und die Erzeugung des dreidimensionalen Stereobildes des Zusatzinstruments (200) auf der Grundlage der Verarbeitungsanweisungen vor der Ausgabe des dreidimensionalen Stereobildes auf der Grundlage der fünften Oberflächendaten (1 00a).

10. System (10) zur Überprüfung vor dem Eingriff nach Anspruch 9, wobei beim Einführen des Verfahrensinstruments in die Öffnung im dreidimensionalen Stereobild des Patienten der Parameter, der sich auf das Verfahrensinstrument bezieht, als die dritten Oberflächendaten (55) eingestellt oder installiert wird, so dass das Verfahrensinstrument vom Ziel getrennt oder in Kontakt mit diesem gebracht wird.

11. System zur Überprüfung vor dem Eingriff (10) nach Anspruch 9 oder 10, wobei eine Positionsbeziehung zwischen dem Ziel und der Öffnung in den vierten Oberflächendaten (53, 553a, 553b, 553c) des Ziels enger ist als eine Positionsbeziehung zwischen einem tatsächlichen Ziel des Kiefers des Patienten und der ausgesparten Öffnung (70, 318, 718).

12. System (10) zur Überprüfung vor dem Eingriff nach Anspruch 11, wobei die ersten Oberflächendaten (52, 552) durch Verschieben charakteristischer Punkte eines dreidimensionalen Bildes der Oberfläche zu einer Seite eines Bildes auf der Grundlage der vierten Oberflächendaten (53, 553a, 553b, 553c) des Ziels in Bezug auf charakteristische Punkte des dreidimensionalen Stereobildes des Kiefers des Patienten festgelegt werden.

13. Das System (10) zur Überprüfung vor dem Verfahren nach Anspruch 9 oder 10, das ferner Folgendes umfasst:
einen dreidimensionalen Drucker (24), der so konfiguriert ist, dass er das Modell (100) auf der Grundlage des dreidimensionalen Stereobildes basierend auf den fünften Oberflächendaten (100a) ausgibt.

14. Ein Programm zur Überprüfung eines zahnärztlichen Behandlungsplans vor der Behandlung, wobei das Programm einen Computer (12) zur Ausführung veranlasst:
ein erstes Verfahren, bei dem in einem dreidimensionalen Stereobild eines Kiefers eines Patienten eine Behandlungszielstelle des Patienten und ein Ziel spezifiziert wird, das mit einem distalen Ende einer Verfahrenseinheit nicht in Kontakt oder in Kontakt sein soll Instrument, das für eine Behandlung am Zielort der Behandlung verwendet wird;
ein zweites Verfahren zum Einstellen einer Öffnung in dem dreidimensionalen Stereobild des Kiefers des Patienten, die eine vertiefte Öffnung (70, 318, 718) zum Einbetten eines eingebetteten Körpers modelliert, der in die Behandlungszielstelle eingebettet werden soll, durch Einstellen oder Installieren mindestens eines Parameters, der sich auf den eingebetteten Körper bezieht, und eines Parameters, der sich auf das Verfahrensinstrument bezieht;
ein drittes Verfahren zur Erzeugung eines dreidimensionalen Stereobildes auf der Grundlage von fünften Oberflächendaten (100a) eines Modells (100) zur Überprüfung des zahnärztlichen Behandlungsplans vor dem Eingriff, wobei die fünften Oberflächendaten (100a) durch Subtraktion von mindestens einer der zweiten Oberflächendaten (54) einer Oberfläche des eingebetteten Körpers und der dritten Oberflächendaten (55) einer Oberfläche des Verfahrensinstruments aus ersten Oberflächendaten (52, 552) der Behandlungszielstelle und einer peripheren Stelle davon erhalten werden, wobei die fünften Oberflächendaten (100a) eine Positionsbeziehung zwischen der Behandlungszielstelle einschließlich der Öffnung und vierten Oberflächendaten (53, 553a, 553b, 553c) des Ziels anzeigen; und
einen vierten Prozess der Ausgabe des dreidimensionalen Stereobildes auf der Grundlage der fünften Oberflächendaten (100a) des Modells (100),
wobei:
das Spezifizieren des Ziels das Spezifizieren eines Bildes einer Hauptsäule (160) umfasst, die mit einem Bild verbunden ist, das auf den ersten Oberflächendaten (52, 552) basiert, wobei das Programm den Computer (12) veranlasst, Folgendes auszuführen: Bilden des Modells (100) auf der Grundlage der fünften Fläche Daten (100a), wobei das Modell (100) umfasst:
einen Modellhauptkörper (110) in einer Größe und Form, die mit mindestens einem Teil des Behandlungsziels identisch ist, und der so konfiguriert ist, dass er den mindestens einen Teil des Behandlungsziels modelliert;
einen eine Öffnung definierenden Abschnitt (130), der in den Modellhauptkörper (110) und so gestaltet, dass er die Öffnung definiert;
und einen Zielmodellabschnitt (120), der so konfiguriert ist, dass er das Ziel modelliert und es dem distalen Ende des Verfahrensinstruments ermöglicht, unter der Bedingung, dass das Verfahrensinstrument durch den die Öffnung definierenden Abschnitt (130) hindurchgeht, nicht in Kontakt mit dem Zielmodellabschnitt (120) zu sein in einer Positionsbeziehung des Zielmodellabschnitts (120), die identisch ist mit einer Positionsbeziehung des Ziels relativ zu der Behandlungszielstelle, oder um zu ermöglichen, dass das distale Ende des Verfahrensinstruments in Kontakt mit dem Zielmodellabschnitt (120) unter einer Bedingung ist, dass das Verfahrensinstrument durch den die Öffnung definierenden Abschnitt (130) in einer Positionsbeziehung des Zielmodellabschnitts (120) näher als die Positionsbeziehung des Ziels relativ zu der Behandlungszielstelle hindurchgeht, wobei das Formen des Modells (100) das Formen einen Raum (145), der zwischen dem die Öffnung definierenden Abschnitt (130) und dem Zielmodellabschnitt (120) ausgebildet ist, um eine visuelle Erkennung eines zwischen dem die Öffnung definierenden Abschnitt (130) und dem Zielmodellabschnitt (120) vorhandenen Bereichs zu ermöglichen, wobei der Bereich einen erreichbaren Bereich des distalen Endes des Verfahrensinstruments von dem die Öffnung definierenden Abschnitt einschließt (130) in Richtung des Zielmodellabschnitts (120) und eines erreichbaren Bereichs des distalen Endes des Verfahrensinstruments von dem die Öffnung definierenden Teil (130) in einer Zahnausrichtungsrichtung und einer die Zahnausrichtungsrichtung schneidenden Richtung.

## Revendications

1. Modèle (100) pour pré-vérification de procédure d'un plan de traitement dentaire, comprenant :
un corps principal de modèle (110) formé sur la base d'une mâchoire d'un patient dans une taille et une forme identique à au moins une partie d'un site objectif de traitement de la mâchoire du patient et configuré pour modéliser l'au moins une partie du site objectif de traitement ;
une section définissant une ouverture (130) disposée dans le corps principal de modèle (110) et configurée pour définir une ouverture correspondant à une ouverture en retrait (70, 318, 718) pour incorporer un corps incorporé dans le site objectif de traitement ; et
une section de modèle cible (120) configurée pour modéliser une cible adjacente à ou incorporée dans un os alvéolaire (312, 712) du site objectif de traitement,
la section de modèle cible (120) permettant à une extrémité distale d'un instrument d'intervention utilisé pour un traitement d'être hors de contact avec la section de modèle cible (120) à condition que l'instrument d'intervention passe à travers la section définissant une ouverture (130) dans une relation positionnelle de la partie de modèle cible (120) identique à une relation positionnelle de la cible par rapport au site objectif de traitement, ou
la section de modèle cible (120) permettant à l'extrémité distale de l'instrument d'intervention d'être en contact avec la section de modèle cible (120) à condition que l'instrument d'intervention passe à travers la section définissant une ouverture (130) dans une relation positionnelle de la section de modèle cible (120) plus étroite que la relation positionnelle de la cible par rapport au site objectif du traitement,
dans lequel :
un espace (145) est formé entre la section définissant une ouverture (130) et la section de modèle cible (120) pour permettre une reconnaissance visuelle d'une étendue présente entre la section définissant une ouverture (130) et la section de modèle cible (120), l'étendue incluant une étendue atteignable de l'extrémité distale de l'instrument d'intervention de la section définissant une ouverture (130) vers la section de modèle cible (120) et une étendue atteignable de l'extrémité distale de l'instrument d'intervention à partir de la section définissant une ouverture (130) dans une direction d'alignement des dents et une direction croisant la direction d'alignement des dents.

2. Modèle (100) selon la revendication 1, dans lequel :
la section de modèle cible (120) est soutenue sur le corps principal de modèle (110) avec un pilier principal (160), et
le pilier principal (160) fait partie intégrante d'un cadre qui définit l'espace (145).

3. Modèle (100) selon la revendication 1 ou 2, dans lequel :
le corps principal de modèle (110) comprend une paroi latérale linguale ou une paroi latérale palatine qui modélise une surface de l'os alvéolaire (312, 712).

4. Modèle (100) selon l'une quelconque des revendications 1 à 3, comprenant :
une section de base (140) qui permet à la section de modèle cible (120) d'être placée entre la section de base (140) et le corps principal de modèle (110) ; et
au moins l'un parmi :
un pilier ou des piliers (150) reliant le corps principal de modèle (110) et la section de base (140) et reliant le corps principal de modèle (110) et la section de modèle cible (120) ;
un pilier ou des piliers (150) reliant le corps principal de modèle (110) et la section de base (140) et reliant la section de base (140) et la section de modèle cible (120) ; et
un pilier ou des piliers (150) reliant le corps principal de modèle (110) et la section de modèle cible (120) et reliant la section de base (140) et la section de modèle cible (120).

5. Instrument de pré-vérification de procédure (260) d'un plan de traitement dentaire, comprenant :
le modèle (100) selon l'une quelconque des revendications 1 à 4 ; et
un instrument complémentaire (200) configuré pour s'adapter dans ou se mettre en prise avec le modèle (100) et pour guider l'instrument d'intervention pour former la section définissant une ouverture (130) dans une position souhaitée.

6. Procédé de fabrication d'un modèle (100) pour pré-vérification de procédure d'un plan de traitement dentaire, comprenant :
le fait d'amener, sur la base d'une instruction par un planificateur de traitement dentaire, un ordinateur (12) à spécifier, sur une image stéréo 3D d'une mâchoire d'un patient, un site objectif de traitement du patient et une cible qui doit être hors de contact ou en contact avec une extrémité distale d'un instrument d'intervention utilisé pour un traitement sur le site objectif de traitement ;
le fait d'amener, sur la base de l'instruction par le planificateur de traitement dentaire, l'ordinateur (12) à déterminer, sur l'image stéréo 3D de la mâchoire du patient, une ouverture qui modélise une ouverture en retrait (70, 318, 718) pour incorporer un corps incorporé devant être incorporé dans le site objectif de traitement en déterminant ou en installant au moins l'un d'un paramètre relatif au corps incorporé et un paramètre relatif à l'instrument d'intervention ; et
le fait d'amener, sur la base de l'instruction par le planificateur de traitement dentaire, l'ordinateur (12) à créer une image stéréo 3D sur la base de cinquièmes données de surface (100a) du modèle (100), les cinquièmes données de surface (100a) étant obtenues en soustrayant au moins l'une de deuxièmes données de surface (54) d'une surface du corps incorporé et de troisièmes données de surface (55) d'une surface de l'instrument d'intervention de premières données de surface (52, 552) du site objectif de traitement et d'un site périphérique de celui-ci, les cinquièmes données de surface (100a) indiquant une relation positionnelle entre le site objectif de traitement incluant l'ouverture et des quatrièmes données de surface (53, 553a, 553b, 553c) de la cible,
dans lequel le fait d'amener l'ordinateur (12) à spécifier la cible inclut la spécification d'une image d'un pilier principal (160) associée à une image sur la base des premières données de surface (52, 552),
le procédé de fabrication comprenant :
la formation du modèle (100) sur la base des cinquièmes données de surface (100a), le modèle (100) comprenant :
un corps principal de modèle (110) dans une taille et une forme identiques à au moins une partie du site objectif de traitement et configuré pour modéliser l'au moins une partie du site objectif de traitement ;
une section définissant une ouverture (130) placée dans le corps principal de modèle (110) et configurée pour définir l'ouverture ; et
une section de modèle cible (120) configurée pour modéliser la cible, et pour permettre à l'extrémité distale de l'instrument d'intervention d'être hors de contact avec la section de modèle cible (120) à condition que l'instrument d'intervention passe à travers la section définissant une ouverture (130) dans une relation positionnelle de la section de modèle cible (120) identique à une relation positionnelle de la cible par rapport au site objectif de traitement, ou pour permettre à l'extrémité distale de l'instrument d'intervention d'être en contact avec la section de modèle cible (120) à condition que l'instrument d'intervention passe à travers la section définissant une ouverture (130) dans une relation positionnelle de la section de modèle cible (120) plus étroite que la relation positionnelle de la cible par rapport au site objectif du traitement,
dans lequel la formation du modèle (100) inclut la formation d'un espace (145) est formé entre la section définissant une ouverture (130) et la section de modèle cible (120) pour permettre la reconnaissance visuelle d'une étendue présente entre la section définissant une ouverture (130) et la section de modèle cible (120), l'étendue incluant une étendue atteignable de l'extrémité distale de l'instrument d'intervention de la section définissant une ouverture (130) vers la section de modèle cible (120) et une étendue atteignable de l'extrémité distale de l'instrument d'intervention à partir de la section définissant une ouverture (130) dans une direction d'alignement des dents et une direction croisant la direction d'alignement des dents.

7. Procédé de fabrication d'un modèle (100) pour pré-vérification de procédure d'un plan de traitement dentaire, comprenant :
le fait d'amener, sur la base d'une instruction par un planificateur de traitement dentaire, un ordinateur (12) à spécifier, sur une image stéréo 3D d'une mâchoire d'un patient, un site objectif de traitement du patient et une cible qui doit être hors de contact ou en contact avec une extrémité distale d'un instrument d'intervention utilisé pour un traitement sur le site objectif de traitement ;
le fait d'amener, sur la base de l'instruction par le planificateur de traitement dentaire, l'ordinateur (12) à déterminer, sur l'image stéréo 3D de la mâchoire du patient, une ouverture qui modélise une ouverture en retrait (70, 318, 718) pour incorporer un corps incorporé devant être incorporé dans le site objectif de traitement en déterminant ou en installant au moins l'un d'un paramètre relatif au corps incorporé et un paramètre relatif à l'instrument d'intervention ; et
le fait d'amener, sur la base de l'instruction par le planificateur de traitement dentaire, l'ordinateur (12) à créer une image stéréo 3D d'un instrument complémentaire (200) sur la base
d'au moins l'un du paramètre relatif au corps incorporé et du paramètre relatif à l'instrument d'intervention et
des premières données de surface (52, 552),
l'instrument complémentaire (200) étant configuré pour s'adapter à un pourtour du site objectif de traitement pour être utilisé et pour guider l'instrument d'intervention pour former l'ouverture en retrait (70, 318, 718) dans une position souhaitée,
le fait d'amener, sur la base de l'instruction par le planificateur de traitement dentaire, l'ordinateur (12) à créer une image stéréo 3D sur la base de cinquièmes données de surface (100a) du modèle (100), les cinquièmes données de surface (100a) étant obtenues en soustrayant au moins l'une de deuxièmes données de surface (54) d'une surface du corps incorporé et de troisièmes données de surface (55) d'une surface de l'instrument d'intervention de premières données de surface (52, 552) du site objectif de traitement et d'un site périphérique de celui-ci, les cinquièmes données de surface (100a) indiquant une relation positionnelle entre le site objectif de traitement incluant l'ouverture et des quatrièmes données de surface (53, 553a, 553b, 553c) de la cible,
dans lequel le fait d'amener l'ordinateur (12) à spécifier la cible inclut la spécification d'une image d'un pilier principal (160) associée à une image sur la base des premières données de surface (52, 552),
dans lequel :
la création de l'image stéréo 3D sur la base des cinquièmes données de surface (100a) inclut la délivrance en sortie, par l'ordinateur (12), de l'image stéréo 3D de l'instrument complémentaire (200) pour la délivrance en sortie de l'instrument complémentaire (200), et
la création de l'image stéréo 3D de l'instrument complémentaire (200) est réalisée avant la délivrance en sortie, par l'ordinateur (12), de l'image stéréo 3D sur la base des cinquièmes données de surface (100a) pour la délivrance en sortie du modèle (100).

8. Procédé de fabrication selon la revendication 6 ou 7, comprenant :
la détermination des premières données de surface (52, 552) en associant des points caractéristiques d'une image 3D de la surface du site objectif de traitement et du site périphérique de celui-ci à des points caractéristiques de l'image stéréo 3D de la mâchoire du patient, ou
la détermination des premières données de surface (52, 552) en déplaçant les points caractéristiques de l'image 3D de la surface vers un côté d'une image sur la base des quatrièmes données de surface (53, 553a, 553b, 553c) de la cible, par rapport aux points caractéristiques de l'image stéréo 3D de la mâchoire du patient.

9. Système de pré-vérification de procédure (10) d'un plan de traitement dentaire, comprenant :
un appareil de commande (12) ; et
une section d'entrée d'instruction (20) qui est commandée par l'appareil de commande (12) et configurée pour recevoir l'entrée d'instructions pour l'appareil de commande (12), les instructions incluant :
une instruction de traitement pour spécifier, sur une image stéréo 3D d'une mâchoire d'un patient, un site objectif de traitement du patient et une cible qui doit être hors de contact ou en contact avec une extrémité distale d'un instrument d'intervention utilisé pour un traitement sur le site objectif de traitement ;
une instruction de traitement pour déterminer, sur l'image stéréo 3D de la mâchoire du patient, une ouverture qui modélise qui modélise une ouverture en retrait (70, 318, 718) pour incorporer un corps incorporé devant être incorporé dans le site objectif de traitement en déterminant ou en installant au moins l'un d'un paramètre relatif au corps incorporé et d'un paramètre relatif à l'instrument d'intervention ; et
une instruction de traitement pour créer une image stéréo 3D sur la base de cinquièmes données de surface (100a) d'un modèle (100) pour une pré-vérification de procédure du plan de traitement dentaire, les cinquièmes données de surface (100a) étant obtenues en soustrayant au moins l'une de deuxièmes données de surface (54) d'une surface du corps incorporé et de troisièmes données de surface (55) d'une surface de l'instrument d'intervention de premières données de surface (52, 552) du site objectif de traitement et d'un site périphérique de celui-ci, les cinquièmes données de surface (100a) indiquant une relation positionnelle entre le site objectif de traitement incluant l'ouverture et des quatrièmes données de surface (53, 553a, 553b, 553c) de la cible,
dans lequel :
l'instruction de traitement pour spécifier la cible inclut une instruction pour spécifier une image d'un pilier principal (160) associée à une image sur la base des premières données de surface (52, 552), et
l'appareil de commande (12) est configuré pour réaliser le traitement sur la base des instructions de traitement, et est configuré pour délivrer en sortie l'image stéréo 3D sur la base des cinquièmes données de surface (100a) pour une délivrance en sortie du modèle (100),
dans lequel :
la section d'entrée d'instructions (20) est configurée pour recevoir l'entrée d'une instruction de traitement pour créer une image stéréo 3D d'un instrument complémentaire (200) sur la base d'au moins l'un du paramètre relatif au corps incorporé et du paramètre relatif à l'instrument d'intervention et des premières données de surface (52, 552), l'instrument complémentaire (200) étant configuré pour s'adapter à un pourtour du site objectif de traitement pour être utilisé et pour guider l'instrument d'intervention pour former l'ouverture en retrait (70, 318, 718) dans une position souhaitée, et
l'appareil de commande (12) est configuré pour réaliser le traitement sur la base d'instructions de traitement, et est configuré pour délivrer en sortie l'image stéréo 3D sur la base des cinquièmes données de surface (100a) pour la délivrance en sortie du modèle (100) et l'image stéréo 3D de l'instrument complémentaire (200) pour la délivrance en sortie de l'instrument complémentaire (200), et la création de l'image stéréo 3D de l'instrument complémentaire (200) sur la base des instructions de traitement est réalisée avant la délivrance en sortie de l'image stéréo 3D sur la base des cinquièmes données de surface (100a).

10. Système de pré-vérification de procédure (10) selon la revendication 9, dans lequel lors de l'insertion de l'instrument d'intervention dans l'ouverture sur l'image stéréo 3D du patient, le paramètre relatif à l'instrument d'intervention est déterminé ou installé en tant que troisièmes données de surface (55) de telle sorte que l'instrument d'intervention est séparé de la cible ou mis en contact avec elle.

11. Système de pré-vérification de procédure (10) selon la revendication 9 ou 10, dans lequel une relation positionnelle entre la cible et l'ouverture dans les quatrièmes données de surface (53, 553a, 553b, 553c) de la cible est plus étroite qu'une relation positionnelle entre une cible réelle de la mâchoire du patient et l'ouverture en retrait (70, 318, 718).

12. Système de pré-vérification de procédure (10) selon la revendication 11, dans lequel les premières données de surface (52, 552) sont déterminées en déplaçant des points caractéristiques d'une image 3D de la surface vers un côté d'une image sur la base des quatrièmes données de surface (53, 553a, 553b, 553c) de la cible, par rapport aux points caractéristiques de l'image stéréo 3D de la mâchoire du patient.

13. Système de pré-vérification de procédure (10) selon la revendication 9 ou 10, comprenant en outre :
une imprimante 3D (24) configurée pour délivrer en sortie le modèle (100) sur la base de l'image stéréo 3D sur la base des cinquièmes données de surface (100a).

14. Programme de pré-vérification de procédure d'un plan de traitement dentaire, le programme amenant un ordinateur (12) à exécuter :
un premier processus de spécification, sur une image stéréo 3D d'une mâchoire d'un patient, d'un site objectif de traitement du patient et d'une cible qui doit être hors de contact ou en contact avec une extrémité distale d'un instrument d'intervention utilisé pour un traitement sur le site objectif de traitement ;
un deuxième processus de détermination, sur l'image stéréo 3D de la mâchoire du patient, d'une ouverture qui modélise une ouverture en retrait (70, 318, 718) pour incorporer un corps incorporé devant être incorporé dans le site objectif de traitement en déterminant ou en installant au moins l'un d'un paramètre relatif au corps incorporé et d'un paramètre relatif à l'instrument d'intervention ;
un troisième processus de création d'une image stéréo 3D sur la base de cinquièmes données de surface (100a) d'un modèle (100) pour une pré-vérification de procédure du plan de traitement dentaire, les cinquièmes données de surface (100a) étant obtenues en soustrayant au moins l'une de deuxièmes données de surface (54) d'une surface du corps incorporé et de troisièmes données de surface (55) d'une surface de l'instrument d'intervention de premières données de surface (52, 552) du site objectif de traitement et d'un site périphérique de celui-ci, les cinquièmes données de surface (100a) indiquant une relation positionnelle entre le site objectif de traitement incluant l'ouverture et des quatrièmes données de surface (53, 553a, 553b, 553c) de la cible ; et
un quatrième processus de délivrance en sortie de l'image stéréo 3D sur la base des cinquièmes données de surface (100a) du modèle (100) ;
dans lequel :
la spécification de la cible inclut la spécification d'une image d'un pilier principal (160) associé à une image sur la base des premières données de surface (52, 552),
le programme amenant l'ordinateur (12) à exécuter :
la formation du modèle (100) sur la base des cinquièmes données de surface (100a), le modèle (100) comprenant :
un corps principal de modèle (110) dans une taille et une forme identiques à au moins une partie du site objectif de traitement et configuré pour modéliser l'au moins une partie du site objectif de traitement ;
une section définissant une ouverture (130) disposée dans le corps principal de modèle (110) et configurée pour définir l'ouverture ; et
une section de modèle cible (120) configurée pour modéliser la cible, et pour permettre à l'extrémité distale de l'instrument d'intervention d'être hors de contact avec la section de modèle cible (120) à condition que l'instrument d'intervention passe à travers la section définissant une ouverture (130) dans une relation positionnelle de la section de modèle cible (120) identique à une relation positionnelle de la cible par rapport au site objectif de traitement, ou pour permettre à l'extrémité distale de l'instrument d'intervention d'être en contact avec la section de modèle cible (120) à condition que l'instrument d'intervention passe à travers la section définissant une ouverture (130) dans une relation positionnelle de la section de modèle cible (120) plus étroite que la relation positionnelle de la cible par rapport au site objectif du traitement,
dans lequel la formation du modèle (100) inclut la formation d'un espace (145) qui est formé entre la section définissant une ouverture (130) et la section de modèle cible (120) pour permettre une reconnaissance visuelle d'une étendue présente entre la section définissant une ouverture (130) et la section de modèle cible (120), l'étendue incluant une étendue atteignable de l'extrémité distale de l'instrument d'intervention de la section définissant une ouverture (130) vers la section de modèle cible (120) et une étendue atteignable de l'extrémité distale de l'instrument d'intervention à partir de la section définissant une ouverture (130) dans une direction d'alignement des dents et une direction croisant la direction d'alignement des dents.
